Europäisches Patentamt

European Patent Office

Office européen des brevets

(19)

(11) **EP 0 873 187 B1**

(12) **EUROPÄISCHE PATENTSCHRIFT**

(45) Veröffentlichungstag und Bekanntmachung des Hinweises auf die Patenterteilung:
**20.06.2001 Patentblatt 2001/25**

(21) Anmeldenummer: **96939093.9**

(22) Anmeldetag: **18.11.1996**

(51) Int Cl.⁷: **B01J 20/26**, A61L 15/60,
C08F 220/04, C08F 220/58,
C08L 33/02, C08L 33/26,
C09K 17/22

(86) Internationale Anmeldenummer:
**PCT/EP96/05073**

(87) Internationale Veröffentlichungsnummer:
**WO 97/18889 (29.05.1997 Gazette 1997/23)**

(54) **MIT UNGESÄTTIGTEN AMINOALKOHOLEN VERNETZTE, WASSERQUELLBARE POLYMERISATE, DEREN HERSTELLUNG UND VERWENDUNG**

WATER-SWELLING POLYMERS CROSS-LINKED WITH UNSATURATED AMINO ALCOHOLS, THE PRODUCTION AND USE OF SAME

POLYMERES GONFLANT DANS L'EAU, RETICULES AVEC DES AMINO-ALCOOLS INSATURES, LEUR PREPARATION ET LEUR UTILISATION

(84) Benannte Vertragsstaaten:
**AT BE CH DE DK ES FI FR GB GR IE IT LI LU MC NL PT SE**

(30) Priorität: **21.11.1995 DE 19543366**

(43) Veröffentlichungstag der Anmeldung:
**28.10.1998 Patentblatt 1998/44**

(73) Patentinhaber: **STOCKHAUSEN GmbH & Co. KG
47805 Krefeld (DE)**

(72) Erfinder:
• **BREITBACH, Ludger
D-46047 Oberhausen (DE)**
• **DAHMEN, Kurt
D-41239 Mönchengladbach (DE)**
• **HOUBEN, Jochen
D-47906 Kempen (DE)**
• **KÜSTER, Erich
D-47803 Krefeld (DE)**

(74) Vertreter: **Klöpsch, Gerald, Dr.-Ing. Patentanwalt
Boehmert & Boehmert
Anwaltssozietät,
Postfach 18 01 62
40568 Düsseldorf (DE)**

(56) Entgegenhaltungen:
**WO-A-93/21237          WO-A-94/09043
WO-A-95/02002**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft superabsorbierende Polymere für wäßrige Flüssigkeiten, Verfahren zu deren Herstellung und deren Verwendung. Die Polymerisate auf Basis von Carboxylatgruppen enthaltenden Monomeren werden durch die Verwendung von Vernetzern auf Basis von ungesättigten Aminoalkoholen erhalten, die aufgrund ihrer Struktur sowohl bei der radikalischen Vorvernetzung als auch bei der thermischen Nachvernetzung aktiv sind. Die vernetzten superabsorbierenden Polymerisate zeigen hervorragende Eingenschaften.

Superabsorbierende Polymere sind wasserunlösliche, vernetzte Polymere, die in der Lage sind, unter Quellung und Ausbildung von Hydrogelen große Mengen an wäßrigen Flüssigkeiten und Körperflüssigkeiten, wie z.B. Urin oder Blut, aufzunehmen und die absorbierte Flüssigkeitsmenge unter einem bestimmten Druck zurückzuhalten. Durch diese charakteristischen Absorptionseigenschaften finden die Polymeren hauptsächlich Anwendung bei der Einarbeitung in Sanitärartikel, wie z.B. in Babywindeln und Damenbinden sowie im Bereich der Pflanzenaufzucht.

Bei den kommerziell verfügbaren superabsorbierenden Polymeren handelt es sich im wesentlichen um vernetzte Polyacrylsäuren oder vernetzte Stärke/Acrylsäure-Pfropfcopolymerisate, bei denen die Carboxylgruppen teilweise mit Natrium- oder Kalium-Ionen neutralisiert sind.

Die Herstellung superabsorbierender Polymere erfolgt überwiegend durch Polymerisation wäßriger Lösungen von Mischungen aus teilneutralisierter Acrylsäure und Vernetzer zu einem Polymergel, das nach mechanischer Zerkleinerung getrocknet und auf eine bestimmte Korngröße gemahlen wird. Alternativ können Polymerpulver auch über eine inverse Suspensionspolymerisation gewonnen werden, bei der die wäßrige Monomerphase in einer Ölphase, die z.B. aus Cyclohexan besteht, mit Hilfsmitteln suspendiert und anschließend polymerisiert wird.

Die Superabsorber können durch das Verfahren der nachträglichen Oberflächenvernetzung in ihrem Eigenschaftsprofil verbessert werden, insbesondere auch in ihrer Flüssigkeitsaufnahme unter Druck, da das bekannte Phänomen des "gel blocking" unterdrückt wird, bei dem angequollene Polymerteilchen miteinander verkleben und eine weitere Flüssigkeitsaufnahme und Flüssigkeitsverteilung in der Windel behindern. Während der Nachvernetzung werden die Carboxylgruppen der Polymermoleküle an der Oberfläche der Superabsorberteilchen mit Vernetzungsmitteln unter erhöhter Temperatur vernetzt. Als Vernetzer finden u.a. mehrwertige Metallsalze, Glycidylverbindungen, Polyole, Polyepoxide, Polyamine, Alkylencarbonate und Polyethylenglykole Verwendung. Der Schritt der Nachvernetzung kann auch mehrfach erfolgen.

Im Zuge der technischen Weiterentwicklung der superabsorbierenden Polymere hat sich das Anforderungsprofil an diese Produkte über die Jahre deutlich verändert. Während in der Entwicklung der Superabsorber zunächst allein das sehr hohe Quellvermögen bei Kontakt mit Flüssigkeit im Vordergrund stand, hat sich später gezeigt, daß es nicht nur auf die Menge der zu absorbierenden Flüssigkeit ankommt, sondern auch auf die Festigkeit des gequollenen Gels. Es muß ein ausgewogenes Verhältnis von Retention und Gelstärke angestrebt werden, damit im Anwendungsfall die Flüssigkeitsaufnahme auch gegen einen ausgeübten Druck erfolgen kann. Diese spezifische Absorptionseigenschaft wird in der EP 339 461 als Aufnahme unter Druck bezeichnet. Die Methode zur Messung der Flüssigkeitsaufnahme unter Druck (AUL) wird bei verschieden hohen Belastungen durchgeführt. Im Zuge der gestiegenen Anforderungen an Superabsorber hat es sich herausgestellt, daß die ursprüngliche Prüfbelastung von 21 $g/cm^2$ (0,3 psi) nicht mehr den erwünschten Eigenschaftsstandard mißt, wie er für Inkontinenzprodukte bzw.für Windelkonstruktionen mit niedrigen Fluffgehalten und hohen Mengen Superabsorber erforderlich ist. Demzufolge werden heute anwendungsbezogene Druckbelastungen auch bei 42 $g/cm^2$ (0.6 psi) und bei 49 $g/cm^2$ (0.7 psi) gemessen.

Neben einem hohen Niveau der Retention und der Flüssigkeitsaufnahme unter Druck müssen Superabsorber niedrige Mengen an löslichen Anteilen enthalten, die aufgrund unvollkommener Vernetzung während der Polymerisationsreaktion auftreten und im Anwendungfall nicht vollständig im Polymerkörper zurückgehalten werden. Dies führt letztlich zu einer Verminderung der Fähigkeit des Superabsorbers zur Flüssigkeitsaufnahme und Flüssigkeitsverteilung in der Windel. Als Grenzwerte für niedrige lösliche Anteile werden z.B. in der US Re. 32,649 7.5 % nach 1 Stunde und 17% nach 16 Stunden angegeben.

Ein weiterer wichtiger Eigenschaftsparameter ist die Permeabilität des gequollenen Superabsorbers für wäßrige Flüssigkeiten. Je höher die Durchlässigkeit des gequollenen Superabsorber für wäßrige Flüssigkeiten ist, desto weniger kann er in Windeln sogenannte Sperrschichten aufbauen, die den Weitertransprot von Flüssigkeiten abblocken. Die Methode zur Bestimmung der Permeabilität ist in der EP 640 330 A1 (Seite 19-21) als "Gel Layer Permeability Test" (GLP) beschrieben.

Die Optimierung der Gebrauchseigenschaften der superabsorbierenden Polymere erfolgte in der Vergangenheit vor allem durch Variation der Vernetzerart und Vernetzermenge, durch den pH-Wert während der Polymerisation und durch Nachbehandlung der Polymerpartikel im Sinne einer Beschichtung und/oder Oberflächennachvernetzung.

In der US Re 32,649 wird durch das Verfahren der sauren Polymerisation eine verbesserte Gelstabilität bei niedrigen löslichen Anteilen erreicht. Das Herstellungsverfahren der Re 32,649 weist wesentliche Verfahrensmängel auf. Zum einen liegt aufgrund der niedrigen Ansatzkonzentration und einer mehrstündigen Nacherhitzung des Polymergels eine unwirtschaftlich niedrige Raum/Zeit-Ausbeute vor, zum anderen ist der Verfahrensschritt der nachträglichen Neutralisation des festen Polymergels technisch sehr zeitaufwendig und nicht in der Qualität durchzuführen, wie es eine Neutralisation in der vorausgehenden Lösung sein kann. Nachträglich neutralisierte Polymergele sind in der Regel nicht gleichmäßig durchneutralisiert und oftmals aufgrund von ungleichmäßiger Alkaliverteilung verfärbt. Als Folge der ungleichmäßigen Neutralisation können auch starke Qualitätsschwankungen innerhalb des Produktes entstehen. Unter einer Vielzahl von möglichen Vernetzern werden Di- und Polyester ungesättigter Mono- oder Polycarbonsäuren mit Polyolen, Bisacrylamide, Di- oder Triallylamine als bevorzugt, und als besonders bevorzugt N,N-Methylenbisacrylamid, Trimethylolpropantriacrylat und Triallylamin genannt.

Der WO 94/09043 liegt die Aufgabestellung zugrunde, neue superabsorbierende Polymere mit einem für wäßrige Flüssigkeiten erhöhten Aufnahmevermögen, auch unter Druckbelastung bereitzustellen. Sie beschreibt als Lösung dieser Aufgabe doppelt vernetzte Superabsorber, deren Herstellung in der ersten Stufe die Vernetzung während der Polymerisation mit Methylenbisacrylamid, Bis(acrylamido)essigsäure, Allylacrylat, Allylmethacrylat, Estern bzw. Amiden mit endständigen Vinyl- und Allyl-Funktionen oder hoch ethoxyliertem Trimethylolpropantriacrylat vorsieht und in einer zweiten Stufe die entstandenen Polymerpartikel an der Oberfläche mit einem Vernetzer beschichtet und dann vernetzt.

Die WO 93/21237 beschreibt superabsorbierende Polymere, die mit ungesättigten Estern von Polyalkylglykolen vernetzt sind und die in einem Nacherhitzungsprozeß eine Eigenschaftsverbesserung bezüglich Retention und Flüssigkeitsaufnahme unter niedrigem Druck 21 $g/cm^2$ ( 0.3 psi) auf jeweils 25 g/g erlangen. Ethoxyliertes Trimethylolpropantriacrylat ist der bevorzugte Vernetzer, wobei die Anzahl der EO-Einheiten pro Polyglykolkette zwischen 2 und 8 liegen kann. Gemäß den Ausführungen in dieser Schrift führt die Verwendung von nicht ethoxyliertem Trimethylolpropantriacrylat zu wesentlich schlechteren Eigenschaften der damit vernetzten Superabsorber.

WO 95/02002 beschreibt pulverförmige, wasserquellbare vernetzte Polymerisate mit hoher Aufnahmekapazität für wässerige Flüssigkeiten, insbesondere Körperflüssigkeiten unter Belastung, die aus mindestens 50 Mol-% neutralisierten säuregruppenhaltigen Monomeren, ggf. copolymerisierbaren Monomeren, einem Vernetzungsmittel und ggf. wasserlöslichen Polymeren gebildet sind. Die Herstellung der Polymerisate erfolgt durch Versetzen der Monomere mit Kohlendioxid als Treibmittel und anschließende Polymerisation, Trocknen und Nachvernetzen mit einem Nachvernetzer. Eine besondere Verbesserung der Absorptionsgeschwindigkeit wird durch Zusatz von Kohlendioxid erzielt.

Aufgabe der Erfindung ist es, neuartige Vernetzer und damit vernetzte Polymerisate sowie ein Verfahren zu ihrer Herstellung zur Verfügung zu stellen, die als Superabsorber zur Verwendung in Windelkonstruktionen oder bei anderen technischen Anwendungen geeignet sind. Darüber hinaus bestand die Aufgabe, durch die neuen vernetzten Strukturen Superabsorber bereitzustellen, deren Eigenschaftsniveau den praxisrelevanten Anforderungen an Retention, Absorption unter Druck [49 $g/cm^2$ (0.7 psi)], Quelldruck und Permeabilität GLP des gequollenen Absorbergels für wäßrige Flüssigkeiten entsprechen.

Diese Aufgabe wird durch die Verwendung von Vernetzern gelöst, die dadurch gekennzeichnet sind, daß sie durch Umsetzung einer Glycidverbindung mit ungesättigten Aminen, beispielsweise Allylaminen, unter Öffnung des Epoxidrings eine Hydroxygruppc ausbilden, die optional einer sich anschließenden Ethoxylierung zur Verfügung steht. Auch andere Reaktionswege zur Herstellung der erfindungsgemäßen Vernetzer sind möglich, etwa die Umsetzung von Aminen mit ungesättigten Glycidverbindungen, beispielsweise (Meth)allylglycidethern oder Glycidyl(meth)acrylaten. Entsprechend der verschiedenen Reaktionsmöglichkeiten zur Herstellung dieser Vernetzerstrukturen, lassen sich die folgenden allgemeinen Formeln I bis IV zur Beschreibung der Vernetzer heranziehen:

**Allgemeine Formel I** für Reaktionsprodukte aus (Meth)allylaminen, Monoglycidylverbindunen und optional Alkylenoxiden:

$$H_o R_3 (NR_1 R_2)_p$$

$R_1, R_2$:    $CHR=CH-CH_2$, H        für o=0 ist p=2

        R:    H, $CH_3$        für o=1 ist p=1

$R_3$:  $R_a-C(H)_c(R_6)-CH_2$

        $R_a$:    monovalent für c=0: H
                divalent für c=1: $CH_2$,        $OCH_2$        $C_{1-6}-O-(CHR-CHR-O)_d$,        d=1-45

                $C_{1-6}-CO-O-(CHR-CHR-O)_d$

$R_6$:   $R_7 H$

   $R_7$:   O, O(CHR-CHR-O)$_n$     n: 1 bis 45,

mit der Maßgabe, daß mindestens 2 Reste $R_1$, $R_2$ CHR = CH - CH$_2$ bedeuten.

**Allgemeine Formel II** für Reaktionsprodukte aus (Meth)allylaminen, Di-, Tri- oder Tetraglycidylverbindungen und optional Alkylenoxiden:

$$R_8(NR_1R_2)_2$$

$R_1, R_2$:   CHR=CH-CH$_2$, H

   R:   H, CH$_3$

$R_8$:   CH$_2$-CH(R$_6$)-CH$_2$-O-R$_4$-CH$_2$-CH(R$_6$)-CH$_2$

   $R_4$:   (CHR-CHR-O)$_m$,     m=1 bis 45
   C$_1$-C$_6$-O,
   $R_5$(O-R$_3$-NR$_1$ R$_2$)$_r$-O

      r=0: bei Verwendung von Diglycidverbindungen
      r=1: bei Verwendung von Triglycidverbindungen
      r=2: bei Verwendung von Tetraglycidverbindungen

         R5: Alkylenrest aus einem Polyol, gegebenenfalls mit einer oder zwei OH-Funktionen, die optional mit bis zu 45 Mol Alkylenoxid umgesetzt sein können.

$R_3$:   CH$_2$-CH(R$_6$)-CH$_2$

   $R_6$:   $R_7 H$

      $R_7$:   O, O(CHR-CHR-O)$_n$     n: 1 bis 45

mit der Maßgabe, daß mindestens 2 Reste $R_1$, $R_2$ CHR = CH - CH$_2$ bedeuten.

**Allgemeine Formel III** für Reaktionsprodukte aus (Meth)allylaminen oder ges. primären Aminen mit (Meth)acrylglycidestern und/oder (Meth)allylglycidethern und optional Alkylenoxiden:

$$(R_9-R_3)_a N(B)_c (R_1)_j (R_2)_k$$

B:      C$_1$-C$_6$-Alkyl     <u>für c=0 gilt:</u>
$R_9$:      CHR=CH-CH$_2$-O,     für a=1 sind j=1 und k=1 CH$_2$=CR-CO-O     für a=2 sind j=1 und k=0
$R_1, R_2$:   CHR=CH-CH$_2$, H     für a=3 sind j und k =0

   R:   H, CH$_3$

$R_3$:   CH$_2$-CH(R$_6$)-CH$_2$     <u>Für c=1 gilt:</u>

   $R_6$:   $R_7 H$     für a=1 sind j=1 und k=0

      $R_7$:   O, O(CHR-CHR-O)$_n$     a=2 sind j und k=0
         n: 1 bis 45

**Allgemeine Formel IV** für Reaktionsprodukte aus Di- und Polyaminen mit (Meth)allylglycidethern und/oder (Meth)acrylglycidestern und optional Alkylenoxiden:

$$R_{11}[N(A)_b\text{-}(R_3\text{-}R_9)_p]_z$$

für b=1 ist p=1

für b=0 ist p=2

z=2,3,4

A:     H, $C_1\text{-}C_6$-Alkyl

$R_3$:     $CH_2\text{-}CH(R_6)\text{-}CH_2$

      $R_6$:     $R_7H$

         $R_7$:     O, $O(CHR\text{-}CHR\text{-}O)_n$       n: 1 bis 45

            R:     H, $CH_3$

$R_9$:   $CHR=CH\text{-}CH_2\text{-}O$,

$$CH_2=CR\text{-}CO\text{-}O$$

R11:     di-, tri- oder tetravalenter Alkylenrest aus der Aminkomponente (gemischt oder einzeln aliphatisch, cyloaliphatisch, aromatisch, heterocyclisch)

Als geeignete Reaktionskomponenten zur Herstellung der erfindungsgemäßen ungesättigten Aminoalkohol-Vernetzer sind beispielsweise zu erwähnen:

Aminkomponenten:

Als geeignete Aminkomponenten für die Umsetzung mit den Glycidverbindungen entsprechend den Formeln I bis IV werden sowohl aliphatische, als auch aromatische, heterocyclische und cycloaliphatische Verbindungen verwendet, beispielsweise Methallylamin, Allylamin, Alkyl-(meth)allylamine, wie beispielsweise Methylallylamin, Methylmethallylamin, Ethylmethallylamin und Ethylallylamin, Methyl-, Ethyl-, Propyl - und Butylylamin, Diallylamin, Dimethallylamin, Anilin, Ethylendiamin, Diethylentriamin, Hexamethylendiamin, Trimethylhexamethylendiamin, Neopentandiamin, 1,2-Propylendiamin, 4,7-Dioxadecan-1,10-diamin, 4,9-Dioxadodecan-1,12-diamin, Polyetherdiamine, Polyalkylenglykoldiamine, 3-Amino-1-methylaminopropan, Bis(3-aminopropyl)methylamin, Isophorondiamin, 4,4'-Diaminodicyclohexylmethan, 1-(2-Aminoethyl)piperazin, o-, m- oder p-Phenylendiamin, 4,4'-Diaminodiphenylmethan, 1,4-Diaminoanthrachinon, 2,4,6-Triamino-1,3,5-triazin und deren Gemische.

Glycidverbindungen: Die erfindungsgemäß zu verwendenden Glycidverbindungen können mono-, di- und polyfunktionell sein. Als monofunktionale Verbindungen werden beispielsweise allein oder in Mischung verwendet: Ethylenglykolmonoglycidether und die zugehörigen $C_1\text{-}C_6$-Alkylether bzw. -ester, Glycidol, Ethylenoxid, Propylenoxid, (Meth)allylglycidether, Polyethylenglykolmonoglycidether und die zugehörigen $C_1\text{-}C_6$-Alkylether bzw. -ester, Vinylglycidether, Glycidyl(meth)acrylate, Glycidyl(meth)allylether oder 1-Halogen-2,3-epoxypropan. Als multifunktionale Glycidether werden beispielsweise Ethylenglykol- bzw. Polyglykoldiglycidether, Glycerin-, Trimethylolpropan- bzw. Pentaerythrit-triglycidether, Polyglycerinpolyglycidether, Sorbitolpolyglycidether bzw. deren Gemische eingesetzt. Die zuvor erwähnten Polyethylenglykolketten der Glycidverbindungen können bis zu 45, vorzugsweise bis zu 20 und besonders bevorzugt bis zu 12 Ethylenglykoleinheiten enthalten.

In einer bevorzugten Ausführungsform werden die erfindungsgemäßen Vernetzer an der freien Hydroxylgruppe alkoxyliert. Die erfindungsgemäßen Alkohole werden zu diesem Zweck beispielsweise mit Ethylen- bzw. Propylenoxid oder deren Gemische umgesetzt. Bevorzugt werden die Alkohole mit Ethylenoxid umgesetzt. Hierdurch kann auch gleichzeitig eine bessere Wasserlöslichkeit des Vernetzers erzielt werden. Pro Hydroxylgruppe werden bis zu 45 Mol EO, bevorzugt bis zu 20 Mol EO und besonders bevorzugt bis zu 12 Mol EO angelagert.

Einige Beispiele für erfindungsgemäße Vemetzer sind Diallylaminoethanol, Diallylaminopolyglykolether, 1,3-Bis(diallylamino)-propanol-2, N,N-Diallylamino-1-amino-3-allyloxy-propanol-2, Polyethylenglykol-di(N,N-diallyl-3-amino-2-Hydroxy-prop-1-yl)ether, Ethylenglykol-di(N,N-diallyl-3-amino-2-hydroxy-prop-1-yl)ether, alkoxyliertes 1,3-Bis(diallylamino)propanol-2, alkoxyliertes 1-Allyloxy-3-(Diallylamino)propanol-2, alkoxylierter Polyethylenglykol-di(N,N-diallyl-3-amino-2-hydroxy-prop-1-yl)ether, alkoxylierter Ethylenglykoldi(N,N-diallyl-3-amino-2-hydroxy-prop-1-yl) ether, N,N-di(allyloxy-2-hydroxy-prop-3-yl)anilin, alkoxyliertes N,N-di(allyloxy-2-hydroxy-prop-3-yl)anilin. Die zuvor erwähnten Polyethylenglykolethereinheiten enthalten vorzugsweise höchstens 45 Mol Ethylenoxid, vorzugsweise höchstens 20 und besonders bevorzugt höchstens 15 Mol Ethylenoxid. In einer weiteren bevorzugten Ausführungsform sind die N-Atome der Vernetzer teilweise oder völlig quaterniert. Die erfindungsgemäß zu verwendenden Vernetzer bzw. deren Gemische werden zu 0,01 bis 3,0 Gew. %, vorzugsweise zu 0,05 bis 1,5 Gew. % und besonders bevorzugt zu 0,1 bis 1,0 Gew. % bezogen auf die Monomeren eingesetzt.

In einer weiteren, bevorzugten Ausführungsform hat sich die Verwendung von Mischungen aus hoch und niedrig alkoxylierten erfindungsgemäß zu verwendenden Vernetzern bei der Vernetzung der erfindungsgemäßen Superabsorber bewährt.

Die erfindungsgemäßen Vernetzer führen überraschenderweise durch die im weiteren Verfahrensablauf der Absorberherstellung auftretenden thermischen Behandlungen, wie z.B. der Geltrocknung zu Nachvernetzungsreaktionen, die das Eigenschaftsprofil der Superabsorber verbessern. Dadurch wird die Möglichkeit eröffnet, die üblicherweise durchgeführte Oberflächen-Nachvernetzung wegzulassen oder zumindest die Menge an Nachvernetzer zu reduzieren. Die Effektivität dieser Sekundärvernetzung, bei der die Carboxylatgruppen des Polymerteilchens miteinander vernetzt werden, kann durch die erfindungsgemäße Ethoxylierung der freien OH-Gruppen noch gesteigert werden.

Zur weiteren Modifizierung der Eigenschaften der Superabsorber können optional andere Covernetzer in Mengen bis zu 1,0 Gew. %, bevorzugt zu 0,01 - 0,8 Gew. % und besonders bevorzugt zu 0,05 - 0,4 Gew. % bezogen auf die Monomere eingesetzt werden. Bevorzugte Covernetzer sind solche, die mindestens zwei ethylenisch ungesättigte Doppelbindungen enthalten, wie z.B. Methylenbisacrylamid bzw. -methacrylamid oder Ethylenbisacrylamid, ferner Ester der ungesättigten Mono- oder Polycarbonsäuren von Polyolen, wie Diacrylate oder Triacrylate, z.B. Butandioloder Ethylenglykoldiacrylat bzw. -methacrylat, Trimethylolpropantriacrylat, sowie deren Alkoxylate, ferner Allylverbindungen wie Allyl(meth)acrylat, Triallylcyanurat, Maleinsäurediallylester, Pollyallylester, Tetraallyloxiethan, Di- und Triallylamin, Tetraallylethylendiamin, Allylester der Phosphorsäure bzw. phosphorigen Säure. Weiterhin sind Verbindungen, die mindestens eine gegenüber Säuregruppen reaktive funktionelle Gruppe besitzen, einsetzbar. Als Beispiel seien die N-Methylolverbindungen von Amiden, wie Methacrylamid bzw. Acrylamid und die davon abgeleiteten Ether, sowie Di- und Polyglycidylverbindungen genannt.

Das erfindungsgemäß zu verwendende, wäßrige Flüssigkeiten absorbierende Polymerisat wird erhalten durch Polymerisation von ethylenisch ungesättigten, Säuregruppen tragenden Monomeren, beispielsweise aus Acrylsäure, Methacrylsäure, Vinylessigsäure, Maleinsäure, 2-Acrylamido-2-methylpropansulfonsäure, Vinylsulfonsäure, (Meth)allylsulfonsäure bzw. deren Gemische in Gegenwart der erfindungsgemäßen Vernetzer. Der Anteil an diesen sauren Monomeren beträgt in der Monomermischung 55 - 99 Gew. % Die sauren Monomeren sind mindestens zu 25 Mol% und bevorzugt zu mindestens 50 Mol.% und besonders bevorzugt zu 50 bis 80 Mol % neutralisiert und liegen dann beispielsweise als Natrium-, Kalium- oder Ammoniumsalz bzw. deren Gemische vor. Die Neutralisation wird entweder durch die Zugabe der entsprechenden Alkali- bzw. Ammoniumhydroxyde oder mit den entsprechenden Carbonaten oder Hydrogencarbonaten ausgeführt.

Optional können die erfindungsgemäßen Polymerisate weitere, in der wäßrigen Monomerlösung weitgehend lösliche Comonomere zur Modifizierung der Eigenschaften enthalten. Solche Comonomere können beispielsweise (Meth)acrylamid, (Meth)acrylnitril, Vinylpyrrolidon, Vinylacetamid, Hydroxyethylacrylat, Alkylpolyethylenglykol(meth) acrylate, Alkylaminoalkyl(meth)-acrylate, Alkylaminopropylacrylamide, Acrylamidopropyltrimethylammoniumchlorid oder deren Gemische sein. Derartige Comonomere sollten einen Anteil von 40 Gew.%, bezogen auf die sauren Monomere, nicht überschreiten, da sie die Quellfähigkeit des Superabsorbers gegebenenfalls beeinträchtigen können.

Die erfindungsgemäßen Polymerisate können wasserlösliche Polymere als Pfropfgrundlage in Mengen bis zu 30 Gew.%, bezogen auf die Menge der vorhandenen Monomere, enthalten. Dazu zählen unter anderem teil- oder vollverseifte Poly-vinylalkohole, Stärke oder Stärkederivate, Cellulose oder Cellulosederivate, Polyacrylsäuren, Polyglykole oder deren Gemische. Die Molekulargewichte der als Pfropfgrundlage zugesetzten Polymere müssen an die Gegebenheiten der Polymerisationsbedingungen angepaßt sein. So kann es z.B. im Falle einer wäßrigen Lösungspolymerisation aus Gründen der Viskosität der Polymerisatlösung erforderlich sein, nur niedrig- oder mittelmolekulare Polymere einzusetzen, während bei der Suspensionspolymerisation dieser Faktor eine untergeordnete Rolle spielt.

Neben Polymerisaten, die durch vernetzende Polymerisation teilneutralisierter Acrylsäure erhalten werden, werden bevorzugt solche verwendet, die zusätzliche Anteile von pfropfpolymerisierter Stärke oder von Polyvinylalkohol enthalten.

Der Zusatz von Natur- und/oder Synthese-Fasern bewirkt eine schnellere Flüssigkeitsaufnahme und eine erhöhte Retention des erfindungsgemäßen Superabsorbers.

Die Herstellung der erfindungsgemäßen Superabsorber geschieht prinzipiell nach zwei Methoden:

Nach der ersten Methode wird die teilneutralisierte Acrylsäure in wäßriger Lösung in Gegenwart der erfindungsgemäßen Vernetzer sowie gegebenenfalls weiterer Vernetzer und Polymerzusätzen durch radikalische Polymerisation in ein Gel überführt, das dann zerkleinert, getrocknet, gemahlen, nachvernetzt und auf die gewünschte Partikelgröße abgesiebt wird. Die Lösungspolymerisation kann kontinuierlich oder diskontinuierlich durch geführt werden. Die Patentliteratur weist sowohl ein breites Spektrum an Variationsmöglichkeiten hinsichtlich der Konzentrationsverhältnisse, Temperaturen, Art und Menge der Initiatoren als auch eine Vielzahl von Nachvernetzungsmöglichkeiten aus. Typische Verfahren sind in den folgenden Patentschriften beschrieben, die hiermit durch Verweis Bestandteil des erfindungsgemäßen Herstellverfahrens werden sollen: US 4 076 663, US 4 286 082, DE 27 06 135, DE 35 03 458, DE 40 20 780, DE 42 44 548, DE 43 23 001, DE 43 33 056, DE 44 18 818.

Die zweite Methode umfaßt das inverse Suspensions- und Emulsionspolymerisationsverfahren. In diesen Prozessen wird eine wäßrige, teilneutralisierte Acrylsäurelösung mit Hilfe von Schutzkolloiden und/oder Emulgatoren in einem hydrophoben, organischen Lösungsmittel dispergiert und durch Radikalinitiatoren die Polymerisation gestartet. Die Vernetzer sind entweder in der Monomerlösung gelöst und werden mit dieser zusammen dosiert oder aber separat und gegebenenfalls nachträglich zugefügt. Die Zugabe von gegebenenfalls vorhandenen polymeren Pfropfgrundlagen erfolgt über die Monomerlösung oder durch direkte Vorlage in die Ölphase. Nach Beendigung der Polymerisation wird das Wasser azeotrop aus dem Reaktionsgemisch entfernt und das Polymerprodukt abfiltriert.

Eine Oberflächenvernetzung der Polymerteilchen kann sowohl in der Suspension als auch nachträglich am isolierten Polymerpulver vorgenommen werden. Das Verfahrensprinzip ist beispielsweise in den Patentschriften US 43 40 706, DE 37 13 601, DE 28 40 010 beschrieben und soll hiermit durch Verweis Bestandteil des erfindungsgemäßen Herstellverfahrens werden.

Als Nachvernetzer geeignet sind insbesondere Polyole, Polyepoxide, Polyamine oder Alkylencarbonate.

Der Zusatz der Nachvernetzer erfolgt häufig vorteilhafterweise auch in Form einer Lösung in Wasser, organischen Lösemitteln oder deren Mischungen, insbesondere dann, wenn geringe Mengen an Nachvernetzungsmittel angewandt werden. Geeignete Mischaggregate zum Aufbringen des Nachvernetzungsmittels sind z.B. Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Pulver mittels rotierender Messer in schneller Frequenz gemischt wird (Schugi-Mischer). Nachdem der Nachvernetzer mit dem vorvernetzten Polymer vermischt worden ist, wird zur Durchführung der Nachvernetzungsreaktion auf Temperaturen von 120 bis 250 °C, bevorzugt auf 135 bis 200°C und besonders bevorzugt auf 150 bis 185°C erhitzt. Die Zeitdauer der Nacherhitzung ist durch den Punkt begrenzt, bei dem das gewünschte Eigenschaftsprofil des Superabsorbers infolge von Hitzeschädigung wieder zerstört wird

Die Oberflächenvernetzung kann bei erhöhter Temperatur und ggf. mehrfach durchgeführt werden.

Aufgrund der besonderen Struktur der erfindungsgemäß zu verwendenden Vernetzer zeigen die Polymerisate bereits ohne Zusatz von Nachvernetzern allein durch Temperung einen für die Nachvernetzung typischen Effekt der Steigerung der Absorption unter Druck.

Die erfindungsgemäß hergestellten Superabsorber zeigen eine bisher noch nicht erreichte Kombination von günstigen Eigenschaften. Aufgrund des positiven Einflusses der erfindungsgemäßen Vernetzer wird die vor der Nachvernetzung vorhandene hohe Retention des Polymers derart gut stabilisiert, daß nach der Oberflächen-Nachvernetzung immer noch eine Retention von mindestens 26 g/g, bevorzugt von mindestens 28 g/g und besonders bevorzugt von mindestens 30 g/g gemessen wird.

Die Permeabilität der erfindungsgemäßen Superabsorber ist hoch und beläuft sich auf $10 \cdot 10^{-7}$ cm$^3$ sec/g, bevorzugt auf mindestens $15 \cdot 10^{-7}$ cm$^3$ sec/g und besonders bevorzugt auf mindestens $25 \cdot 10^{-7}$ cm$^3$ sec/g.

Nach erfolgter Flüssigkeitsaufnahme, deren Geschwindigkeit vorzugsweise bei kleiner 70 sec liegt, zeichnen sich die gequollenen Gelpartikel durch einen trocknen Griff aus, d.h. sie haben nicht die unerwünschte nasse, klebrige Oberfläche, die bei unzureichender Vernetzung/Nachvernetzung entsteht. Die Flüssigkeitsaufnahme unter einem Druck (AUL) von 49 g/cm$^2$ (0.7 psi) ist größer 20 g/g, bevorzugt größer 23 g/g und besonders bevorzugt größer 25 g/g.

Der Quelldruck der erfindungsgemäßen Polymerisate ist hoch und beträgt nach einer Meßzeit von 20 Minuten mindestens 600 g, bevorzugt mindestens 900 g, und besonders bevorzugt größer 1000g.

Die erfindungsgemäßen hydrophilen Superabsorber finden überall dort ihre Verwendung, wo wäßrige Flüssigkeiten absorbiert werden müssen. Dazu gehören beispielsweise die Anwendungen in Hygieneartikeln in Form von Windeln für Kleinkinder und Inkontinenzprodukten für Erwachsene, in Damenbinden, in Wundpflastern, in Lebensmittelverpackungen, im Agrarbereich bei der Pflanzenaufzucht, in Kabelisolierungen, in absorbierenden Flächengebilden aus Papier, wasserlöslichen Polymeren und thermoplastischen Kunststoffen und Schäumen, sowie als Wirkstoffträger mit der Aufgabe der zeitlich verzögerten Freisetzung an die Umgebung.

Für die Verarbeitung der Superabsorber werden je nach Anwendungsfall unterschiedliche Siebfraktionen eingesetzt, so z.B. für Windeln zwischen 100 und 800μ, für Kabelisolierungen unter 100 μ, d. h., im Falle der Anwendung in Kabeln sind die Feinanteile der Superabsorber wegen ihrer Tendenz zum "gel blocking" von Vorteil, da dadurch das in das Kabel eindringende Wasser abgeblockt wird. In der Windel ist dieser Effekt unerwünscht, da er die Flüssigkeitsaufnahme und Verteilung behindert, deshalb wählt man gröbere Siebfraktionen aus.

In den folgenden Beispielen werden die Herstellung und Eigenschaften der erfindungsgemäßen Polymerisate erläutert und in dem Kapitel Prüfmethoden werden die Vorschriften zur Bestimmung der Eigenschaften der Superabsorber beschrieben.

**Prüfmethoden**

**1. Retention(TBT)**

Die Retention wird nach der Teebeutelmethode bestimmt. Hierbei werden 200 mg Prüfsubstanz in einem Teebeutel eingeschweißt und für 30 Minuten in einer 0,9%igen NaCl-Lösung eingetaucht, 10 Minuten abgetropft und in einer Schleuder (23 cm Durchmesser, 1400 UPM) 5 Minuten geschleudert und gewogen. Einen Teebeutel ohne wasserabsorbierendes Polymerisat läßt man als sogenannten Blindwert mitlaufen:

$$TBT = (Auswaage - Blindwert) / Einwaage\ (g/g)$$

**2. Flüssigkeitsaufnahme unter Druck (AAP)**

Die Fähigkeit eines wasserabsorbierenden Polymerisates unter einem definiertem Druck Flüssigkeit aus einem Reservoir aufzunehmen (Absorption Against Pressure(0,3 psi = 21 g/cm$^2$), AAP(0,7 psi = 49 g/cm$^2$), wird wie folgt bestimmt: 900 mg Prüfsubstanz werden in einem Plastikzylinder (Innendurchmesser = 6 cm, Höhe = 5 cm) mit Siebgewebe (Maschenweite = 400 mesh) als Boden eingewogen, gleichmäßig verteilt und mit einem definiertem Gewicht in Form einer Kunststoffplatte (Durchmesser = 5,98 cm) zusammen mit einem Metallstempel (Durchmesser = 5,98 cm) belastet. Die Kunststoffplatte liegt zwischen Prüfsubstanz und Metallstempel. Die ganze Prüfeinheit wird anschließend auf eine mit einem Filterpapier abgedeckte und mit 0,9%iger NaCl-Lösung getränkte Glasfilterplatte (Durchmesser = 12 cm, Porosität = 0) gestellt. Die Filterplatte liegt bis zu ihrer Oberkante in der NaCl-Lösung. Man läßt die Prüfsubstanz 60 Minuten Flüssigkeit aufnehmen:

$$AAP(0,3\ bzw.\ 0,7\ psi) = (Gewicht\ der\ Prüfeinheit\ vor\ dem\ Saugen - Gewicht\ der\ Prüfeinheit.nach$$

$$dem\ Saugen) / Einwaage\ Prüfsubstanz\ (g/g)$$

**3. Quelldruck (QD)**

Der Quelldruck wird in einem Stevens-LFRA Texture Analyser (Einstellung: Speed: 1,0 mm/sec; Distance 00, Hold-Stellung) bestimmt. Dazu werden in einem Messzylinder von 7,4 cm Höhe und 2,7 cm Durchmesser 0,500 g des Pulvers (Kornfraktion 300 - 600 mm) eingewogen und mit 10 ml 0,9%-iger Kochsalzlösung versetzt. Sodann wird der Messkörper (Höhe 3,5 cm, Durchmesser 2,5 cm) in den Zylinder so eingefahren, daß ein Hohlraum zur Gelquellung von 6,8 ml verbleibt. Der Quelldruck QD wird nach 20 Minuten gemessen.

**4. Geschwindigkeit der Flüssigkeitsaufnahme (SG)**

Bei diesem Test wird die Zeit gemessen, in der 1 g Superabsorber 20g einer 0,9%igen Kochsalzlösung bei Raumtemperatur aufsaugt. Der Ablauf dieser Prüfung ist in der EP 443 627, Seite12, "Free-Swell-Rate", geschildert.

**5. Permeabilität (GLP)**

Die Permeabiltät einer gequollenen Gelschicht unter Druckbelastung von 0,3 psi wird, wie in der Patentschrift EP 0 640 330 beschrieben, als Gel-Layer-Permeability (GLP) einer gequollenen Gelschicht aus superabsorierenden Polymerisat wie folgt bestimmt.

Hierzu wird eine Prüfeinheit verwendet, die sich von der oben beschriebenen AAP(0,3psi)-Prüfeinheit in folgemdem unterscheidet. Anstelle der zwischen Prüfsubstanz und Metallstempel befindlichen Kunstoffplatte wird eine Glasfilterplatte (Durchmesser = 5,98 cm, Porosität = 0) und eine aus Kunststoff hergestellte Lochplatte (Durchmesser = 5,98 cm, 24 Löcher), die in ihrem Mittelpunkt konzentrisch einen Zylinder (Länge = 7 cm, Durchmesser = 1,8 cm) als Abstandshalter zum aufzusetzendem Gewicht besitzt, verwendet. Zur Bestimmung werden 900 mg Prüfsubstanz in einem wie oben beschriebenen Plastikzylinder mit Siebgewebe als Boden eingewogen, gleichmäßig verteilt und mit einem definiertem Gewicht in Form der Glasfilterplatte, der mit einem Abstandshalter versehenen Lochplatte und einem auf dem Abbstandshalter aufgesetzten Metallstempel belastet. Die ganze Prüfeinheit wird anschließend auf eine mit einem Filterpapier abgedeckte und mit synthetischer Urin-Lösung getränkte Glasfilterplatte (Durchmesser = 12 cm, Porosität = 0) gestellt. Die Filterplatte liegt bis zu ihrer Oberkante in der synthetischen Urin-Lösung. Man läßt die Prüfsubstanz 60 Minuten Flüssigkeit aufnehmen. Danach wird die Prüfeinheit von der synthetischen Urin-Lösung entfernt. Danach wird die Gelschicht mit 0,69%iger NaCl-Lösung soweit überschichtet, daß zwischen der Unterkante der Gelschicht und dem Flüssigkeitspegel ein Abstand von 5 cm und somit ein bestimmter hydrostatischer Druck vorliegt. Während der ganzen Messung wird dieser Druck durch eine entsprechende Vorrichtung beibehalten. Der Durchfluß (g-NaCl-Lösung/sec)wird automatisch in bestimmten Zeitintervallen erfaßt.

$$GLP = (F_g(t=0) \cdot L_o)/(d \cdot A \cdot WP) \ (cm^3 \cdot sec/g)$$

wobei $F_g$ (t=0) den Durchfluß der NaCl-Lösung in g/sec, der anhand einer Regressionsanalyse der Daten $F_g(t)$der Durchflußbestimmungen durch Extrapolation gegen t=0 erhalten wird, $L_o$ die Dicke der Gelschicht in cm, d die Dichte der NaCl-Lösung in $g/cm^3$, A die Fläche der Gelschicht in $cm^2$ und WP den hydrostatischen Druck über der Gelschicht in $dyne/cm^2$ darstellt.

**Beispiele**

Herstellungsbeispiele für erfindungsgemäße Vernetzer:

**Beispiel 1:** 1,3-Bis-(diallylamino)-propanol-2 (TAAP)

Die Synthese ist in der Patentschrift FR 2063686 beschrieben.

**Beispiel 2a:**

Umsetzung von Polyethylenglykol(400)diglycidether mit Diallylamin =Polyethylenglykol-400-di(N,N-diallyl-3-amino-2-Hydroxy-prop-1-yl)ether **(PEG400DAAHPE)**

Unter Rühren versetzt man 790 g (1,5 Mol) Polyethylenglykol (400) diglycidether (in 200 mL Aethylacetat mit 2,1 g Diazabicyclo[2.2.2]octan als Katalysator und 291,5 g (3,0 Mol) Diallylamin. Man erhitzt die Lösung 5 h zum Rückfluß (ca. 100°C Innentemperatur) und zieht flüchtige Bestandteile am Rotationsverdampfer im Wasserstrahlvakuum ab. Man erhält eine rötlich-braune Flüssigkeit, die im Kühlschrank fest wird.

Ausbeute:     1067 g (ca. 99 % d. Th.)

**Beispiel 2b:**

Umsetzung von Ethylenglykoldiglycidether mit Diallylamin = Ethylenglykol-di(N,N-diallyl-3-amino-2-hydroxy-prop-1-yl)ether **(EGDAAHPE)**

Unter Rühren versetzt man 260 g Ethylenglykoldiglycidether in 200 ml Ethylacetat mit 2,1 g Diazabicyclo [2.2.2]octan als Katalysator und 291,5 g Diallylamin. Man erhitzt die Lösung 5 h zum Rückfluß (ca. 90°C Innentemperatur) und zieht danach flüchtige Bestandteile am Rotationsverdampfer im Wasserstrahlvakuum ab. Man erhält eine dunkelbraune Flüssigkeit.

Ausbeute:     526 g (ca. 95 % d. Th.)

**Beispiel 3:** 1-Allyloxy-2-hydroxy-3-N,N-diallylamino-propan **(ADAAP)**

Die Synthese ist in der Patentschrift FR 2063686 beschrieben.

**Beispiel 4:** 5-EO-1,3-Bis(diallylamino)propanol-2 **(TAAP-5EO)**

250 g 1,3-Bisdiallylaminopropanol 2 (1 mol) wurden zusammen mit 3 g KOH-Pulver (85% Trockensubstanz) vorgelegt und der Reaktorinhalt während des Aufheizens fünfmal durch Aufdrücken von 5 bar Stickstoff und Ablassen auf Normaldruck inertisiert. Anschließend wurden bei 110°C 220 g (5 mol) Ethylenoxid bis zu einem Druck von 4 bar aufgedrückt. Nach 2 Stunden war die Reaktion beendet und der Reaktorinhalt wurde als gelbes Öl (468 g) ausgefüllt.

Hydroxylzahl:     168 mg KOH/g

**Beispiel 5:.** 10-EO-1,3-Bis(diallylamino)propanol-2 **(TAAP-10EO)**

250 g 1,3-Bisdiallylaminopropanol-2 (1 mol) wurden zusammen mit 3 g KOH-Pulver (85% Trockensubstanz) vorgelegt und der Reaktorinhalt während des Aufheizens fünfmal durch Aufdrücken von 5 bar Stickstoff und Ablassen auf Normaldruck inertisiert. Anschließend wurden bei 110°C 440 g (10 mol) Ethylenoxid bis zu einem Druck von 4 bar aufgedrückt. Nach 2 Stunden war die Reaktion beendet und der Reaktorinhalt wurde als gelbes Öl (786 g) ausgefüllt.

Hydroxylzahl: 112 mg KOH/g

**Beispiel 6:.** 10-EO-1-Allyloxy-3-(Diallylamino)propanol-2 **(ADAAP-10EO)**

211 g 1-Allyloxy-3-(Diallylamino)propanol-2 (1 mol) wurden zusammen mit 3 g KOH-Pulver (85% Trockensubstanz) vorgelegt und der Reaktorinhalt während des Aufheizens fünfmal durch Aufdrücken von 5 bar Stickstoff und Ablassen auf Normaldruck inertisiert. Anschließend wurden bei 110°C 440 g (10 mol) Ethylenoxid bis zu einem Druck von 4 bar aufgedrückt. Nach 2 Stunden war die Reaktion beendet und der Reaktorinhalt wurde als gelbes Öl (650 g) ausgefüllt.

Hydroxylzahl: 118 mg KOH/g

**Beispiel 7:.** 10-EO-Polyethylenglykol-400-di(N,N-diallyl-3-amino-2-hydroxy-prop-1-yl)ether **(PEG400DAAHPE-10EO)**

361 g Polyethylenglykol-400-di(N,N-diallyl-3-amino-2-hydroxy-prop-1-yl)ether **(PEG400DAAHPE;** 0,5 mol) wurden zusammen mit 3 g KOH-Pulver (85% Trockensubstanz) vorgelegt und der Reaktorinhalt während des Aufheizens fünfmal durch Aufdrücken von 5 bar Stickstoff und Ablassen auf Normaldruck inertisiert. Anschließend wurden bei 110°C 220 g (5 mol) Ethylenoxid bis zu einem Druck von 4 bar aufgedrückt. Nach 3 Stunden war die Reaktion beendet und der Reaktorinhalt wurde als gelbes Öl (580 g) ausgefüllt.

Hydroxylzahl: 64 mg KOH/g

**Beispiel 8:** 20-EO- Polyethylenglykol-400-di(N,N-diallyl-3-amino-2-Hydroxy-prop-1-yl)ether **(PEG400DAAHPE-20EO)**

361 g Polyethylenglykol-400-di(N,N-diallyl-3-amino-2-hydroxy-prop-1-yl)ether **(PEG400DAAHPE;** 0,5 mol) wurden zusammen mit 3 g KOH-Pulver (85% Trockensubstanz) vorgelegt und der Reaktorinhalt während des Aufheizens fünfmal durch Aufdrücken von 5 bar Stickstoff und Ablassen auf Normaldruck inertisiert. Anschließend wurden bei 110°C 440 g (10 mol) Ethylenoxid bis zu einem Druck von 4 bar aufgedrückt. Nach 3,5 Stunden war die Reaktion beendet und der Reaktorinhalt wurde als gelbes Öl (802 g) ausgefüllt.

Hydroxylzahl: 46 mg KOH/g

**Beispiel 9:** 10-EO-Ethylenglykoldi(N,N-diallyl-3-amino-2-hydroxy-prop-1-yl)ether **(EGDAAHPE-10EO)**

369 g Ethylenglykoldi(N,N-diallyl-3-amino-2-Hydroxy-prop-1-yl)ether (**EGDAAHPE**; 1,0 mol) wurden zusammen mit 3 g KOH-Pulver (85% Trockensubstanz) vorgelegt und der Reaktorinhalt während des Aufheizens fünfmal durch Aufdrücken von 5 bar Stickstoff und Ablassen auf Normaldruck inertisiert. Anschließend wurden bei 110°C 440 g (10 mol) Ethylenoxid bis zu einem Druck von 4 bar aufgedrückt. Nach 3 Stunden war die Reaktion beendet und der Reaktorinhalt wurde als gelbes Öl (809 g) ausgefüllt.

Hydroxylzahl: 94 mg KOH/g

Herstellungsbeispiele für Superabsorber

Die wasserabsorbierenden Polymerisate, die nach den in den Beispielen aufgeführten Beschreibungen hergestellt wurden, sind nach dem bekannten Verfahren der Lösungspolymerisation hergestellt und mit Ethylencarbonat nach dem in der Patentschrift DE 40 20 780 beschriebenen Verfahren nachvernetzt. Alle %-Angaben sind Gew.-%.

**Beispiel 10:**

Eine wäßrige Acrylsäurelösung, die 0,15% **TAAP**, bezogen auf Acrylsäure, enthält, wurde unter Kühlung mit Natronlauge (50%) neutralisiert. Die Acrylsäurekonzentration der Monomerlösung betrug 30% und der Neutralisationsgrad 70 mol%. 853,2 g der Monomerlösung wurden auf 5°C gekühlt und 10 Minuten mit Stickstoff gespült. Anschließend wurden nacheinander 177 mg tert. Butylhydroperoxid(70%ig) (gelöst in 10 g Wasser), 500 mg Natriumperoxodisulfat (gelöst in 8 g Wasser), 177 mg 2,2'-Azobis-(isobutyronitril)) (gelöst in 10 g Wasser) und 8 mg Ascorbinsäure (gelöst in 2g Wasser) zugemischt. Die Polymerisation beginnt sofort und die entstehende Gelmasse erwärmt sich auf ca 100°C. Nach 30 Minuten wurde der Gelblock zerkleinert und im Heißlufttrockenschrank bei 150°C über 2 Stunden getrocknet. Im Anschluß daran wurde das Polymer gemahlen und auf eine Kornfraktion von 150 bis 850 μm abgesiebt.

Das pulverförmige Polymerisat wurde mit einer Lösung bestehend aus 0,5% Ethylencarbonat/2% Wasser/ 4% Ethanol, bezogen auf Polymerisat, durch intensives Mischen beschichtet und für 20, 30 bzw. 40 Minuten bei 180°C im Ofen erhitzt.

**Beispiele 11 -29:**

Diese Beispiele sind entsprechend dem Beispiel 10 hergestellt worden; nur mit der Änderung, daß andere Vernetzer-Konzentrationen und Typen eingesetzt wurden. Zusammensetzungen und Eigenschaften sind in den Tabellen 2 und 3 angegeben. Die Abkürzung **TMPTA** steht für Trimethylolpropantriacrylat.

**Beispiel 30 - 32:**

Hier soll die nachvernetzende Wirkung der erfindungsgemäßen Vorvernetzer bei nachträglicher Temperung gezeigt werden. Dazu wurden die nicht nachvernetzten Vorprodukte aus Beispiel 14, 22 und 26 einer 30 minütigen Temperaturbehandlung bei 180°C unterzogen und danach hinsichtlich Retention und AAP 21 untersucht. Es zeigte sich ein deutlicher Eigenschaftssprung, wie er für eine Nachvernetzung typisch ist. Die Ergebnisse sind in der Tabelle 4 dargestellt.

**Vergleichsbeispiel 1:**

Es wurden Handelsprodukte hinsichtlich ihrer Retentions- und Permeabilitätseigenschaften untersucht. Die Permeabiltäten sind unabhängig von der Retention durchweg gering.

Tabelle 1:

| Hersteller / Produkt | Retention (g/g) | GLP ($10^{-7}$ cm$^3$ sec/g) |
|---|---|---|
| Atochem / AK 94 EX006 | 30,5 | 0 |
| Allied Colloids / CKH 5164A | 39,5 | 1 |
| Allied Colloids / Salsorb CL20 | 30,5 | 6 |
| Chemdal/ASAP 2000 | 31,0 | 6 |
| Hoechst / IM 7000 | 35,0 | 0 |
| Mitsubishi / US 50 | 34,5 | 3 |
| BASF / Aqualic LF76 | 31,0 | 1 |

**Beispiel 33-38**

Es wird nach der folgenden Richtrezeptur polymerisiert, die Nachvernetzung erfolgt wie in den Beispielen 10 - 29 angegeben:

In einem zylindrischen Kunststoffgefäß wird ein Polymerisationsansatz von insgesamt 1000g zubereitet. Dazu werden 280 g Acrylsäure sowie die verwendeten Vernetzer, Comonomere und weitere Komponenten in vollentsalztem Wasser angesetzt. Unter Rühren und Kühlen wird mit 50%-iger Natronlauge bis zu einem Neutralisationsgrad von 70 % teilneutralisiert. Die Lösung wird auf 7-8°C abgekühlt und mit Stickstoff solange durchperlt, bis der Sauerstoffgehalt in der Monomerenlösung auf einen Wert von unter 0,2 ppm abgesunken ist. Anschließend gibt 100 ppm Azo-bis( 2-amodinopropan)dihydrochlorid gelöst in 10 g VE-Wasser, 300 ppm Natriumpersulfat, gelöst in 6 g VE-Wasser, 70 ppm Wasserstoffperoxid (35%-ig) gelöst in 1 g VE-Wasser hinzu. Danach wird die Polymerisation durch Zugabe von 9 ppm Ascorbinsäure gelöst in 2g Wasser gestartet, worauf eine deutliche Temperaturerhöhung eintritt. Nach Beendigung der Polymerisation wird der gelartige Polymerblock zerkleinert, gewölft und getrocknet. Das Polymer wird anschließend gemahlen und auf die Kornfraktion 150 - 800 μ abgesiebt.

Die Zusammensetzung der Superabsober bezüglich der Vernetzer sowie die Produkteigenschaften sind in der Tabelle 5 aufgeführt.

**Tabelle 2:**

| Bsp-Nr. | Vernetzer | | TBT(Ret) (g/g) | | | | AAP (g/g) | | GLP |
|---|---|---|---|---|---|---|---|---|---|
| | Typ | Gew. % | Vorprod. | nachvern. (180°/20') | nachvern. (180°/30') | nachvern. (180°/40') | 21 g/cm² | 49 g/cm² | (10⁻⁷ cm³sec/g) |
| 10 | TAAP | 0,15 | 38,1 | 32,4 | 30,1 | 31,4 | 28,2 | 22.9 | 30 |
| 11 | TAAP/TMPTA | 0,15/0,05 | 36,7 | 32,6 | 31,7 | 30,8 | 30,2 | 23,2 | 16 |
| 12 | TAAP-5EO | 0,28 | 41,6 | 32,1 | 30,6 | 30 | 31 | 25 | 26 |
| 13 | TAAP-5EO/TMPTA | 0,28/ 0,05 | 40,1 | 31,6 | 30,3 | 29,6 | 32,5 | 26,6 | 14 |
| 14 | TAAP-10EO | 0,41 | 39,2 | 31 | 29,3 | 29,3 | 30,1 | 25,5 | 29 |
| 15 | TAAP-10EO/TMPTA | 0,41/0,05 | 39,2 | 31,3 | 29,8 | 28,4 | 31,3 | 25,6 | 27 |
| 16 | ADAAP | 0,225 | 33,9 | 30 | 29 | 29 | 30,9 | 23,9 | 27 |
| 17 | ADAAP/TMPTA | 0,225/0,05 | 34 | 30,3 | 29,6 | 28,6 | 31,1 | 24,9 | 11 |
| 18 | ADAAP-10EO | 0,225 | 48,5 | 37,4 | 33,8 | 33,1 | 29,7 | 21,0 | 12 |
| 19 | ADAAP-10EO/TMPTA | 0,225/0,05 | 43,0 | 33,9 | 30,9 | 30,6 | 29,8 | 23,3 | 6 |
| 20 | EGDAAHPE | 0,2 | 36,6 | 29,4 | 28,6 | 28,3 | 29,1 | 23,4 | 20 |
| 21 | EGDAAHPE/TMPTA | 0,2/0,05 | 36,5 | 31,7 | 30,3 | 30,3 | 31,7 | 24,9 | 10 |
| 22 | EGDAAHPE-10EO | 0,44 | 39,6 | 28,5 | 26,2 | 25,4 | 22,9 | 19,1 | 30 |
| 23 | EGDAAHPE-10EO/TMPTA | 0,44/0,05 | 36,2 | 27,9 | 28,3 | 26,2 | 29,0 | 24,0 | 29 |
| 24 | PEG400DAAHPE | 0,4 | 36,6 | 30,7 | 30,2 | 28,8 | 30,3 | 23,6 | 16 |
| 25 | PEG400DAAHPE/TMPTA | 0,4/0,05 | 33,9 | 29 | 28,5 | 26,8 | 29,6 | 24,6 | 23 |

EP 0 873 187 B1

| Bsp-Nr. | Vernetzer | | TBT(Ret) (g/g) | | | | AAP (g/g) | | GLP |
|---|---|---|---|---|---|---|---|---|---|
| | Typ | Gew. % | Vorprod. | nachvern. (180°/20') | nachvern.. (180°/30') | nachvern. (180°/40') | 21 g/cm$^2$ | 49 g/cm$^2$ | (10$^{-7}$ cm$^3$sec/g) |
| 26 | PEG400DAAHPE-10EO | 0,61 | 38,0 | 28,8 | 26,9 | 25,6 | 24,3 | 21,0 | 14 |
| 27 | PEG400DAAHPE-10EO/TMPTA | 0,61/0,05 | 36,4 | 29,2 | 27,9 | 26,3 | 24,6 | 21,0 | 26 |
| 28 | PEG400DAAHPE-20EO | 0,61 | 49,5 | 33,7 | 32,1 | 29,2 | 29,7 | 21,8 | 9 |
| 29 | PEG400DAAHPE-20EO/TMPTA | 0,61/0,05 | 42,1 | 31,5 | 29,7 | 30,8 | 28,2 | 22,6 | 20 |

**Tabelle 3:**

| Beispiel | TBT (g/g) | AAP (21 g/cm2) (g/g) | AAP (49 g/cm2) (g/g) | SG (sec) | QD (20') (g) | QD (120') (g) |
|---|---|---|---|---|---|---|
| 10 | 30,1 | 28,2 | 22,9 | | 1110 | 695 |
| 11 | 31,7 | 30,2 | 23,2 | | 960 | 670 |
| 12 | 30,6 | 31,0 | 25,0 | | 1240 | 810 |
| 13 | 30,3 | 32,5 | 26,6 | 65 | 1250 | 875 |
| 14 | 29,3 | 30,1 | 25,5 | | 1250 | 925 |
| 15 | 29,8 | 31,3 | 25,6 | | 1440 | 905 |
| 16 | 29,0 | 30,9 | 23,9 | 74 | 1000 | 650 |
| 17 | 29,6 | 31,1 | 24,9 | | 830 | 580 |
| 18 | 33,8 | 29,7 | 21,0 | | 1050 | 710 |
| 19 | 30,9 | 29,8 | 23,3 | | 1440 | 730 |
| 20 | 28,6 | 29,1 | 23,4 | 62 | 975 | 695 |
| 21 | 30,3 | 31,7 | 24,9 | 60 | 930 | 665 |
| 22 | 26,2 | 22,9 | 19,1 | | 1025 | 710 |
| 23 | 28,3 | 29,0 | 24,0 | | 950 | 660 |
| 24 | 30,2 | 30,3 | 23,6 | 65 | 840 | 600 |
| 25 | 28,5 | 29,6 | 24,6 | 63 | 1025 | 780 |
| 26 | 26,9 | 24,3 | 21,0 | 55 | 1020 | 715 |
| 27 | 27,9 | 24,6 | 21,0 | | 960 | 600 |
| 28 | 32,1 | 29,7 | 21,8 | 61 | 1125 | 680 |

EP 0 873 187 B1

**Tabelle 4:**

| Beispiel | Vorprodukt-Bsp. | Vorprodukt-TBT (g/g) | Vorprodukt-AAP 21 (g/g) | Nacherhitzt-TBT (g/g) | Nacherhitzt-AAP 21 (g/g) |
|---|---|---|---|---|---|
| 30 | 14 | 39,2 | 9,0 | 33.9 | 18,1 |
| 31 | 22 | 39,6 | 7,8 | 29,4 | 20,8 |
| 32 | 26 | 38,0 | 8,4 | 27,2 | 21,9 |

**Tabelle 5:**

| Beispiel | Vernetzertyp (%/Monomer) | Vorprodukt-TBT | Nachvernetzt-TBT | Nachvernetzt-AAP (21 g/cm2) | Nachvernetzt-AAP (49 g/cm2) |
|---|---|---|---|---|---|
| 33 | 0,15/ADAAP-10EO | 39,7 | 29,9 | 30,4 | 23,6 |
| 34 | 0,61/PEG400DAAHPE-10EO | 31,8 | 24,2 | 25,7 | 21,7 |
| 35 | 0,61/PEG400DAAHPE-20EO | 40,2 | 28,1 | 29,5 | 24,1 |
| 36 | 0,61/EGDAAHPE-10EO | 31,6 | 24,5 | 26,2 | 21,9 |
| 37 | 0,15/TAAP | 32,7 | 28,0 | 29,5 | 22,7 |
| 38 | 0,15/TAAP-10EO | 32,1 | 26,9 | 28,5 | 23,3 |

EP 0 873 187 B1

**EP 0 873 187 B1**

**Patentansprüche**

1. Wäßrige Flüssigkeiten absorbierendes vernetztes Polymerisat, aufgebaut aus teilneutralisierten monoethylenisch ungesättigten Säuregruppen tragenden Monomeren, gegebenenfalls weiteren, damit copolymerisierbaren Monomeren sowie gegebenenfalls als Pfropfgrundlage geeigneten Polymeren, dadurch gekennzeichnet, daß es unter Verwendung von Vernetzern aus mehrfach ungesättigten Aminoalkoholen herstellbar ist.

2. Polymerisat nach Anspruch 1, dadurch gekennzeichnet, daß die Vernetzer Umsetzungsprodukte aus (Meth)allylaminen mit Monoglycidylverbindungen und gegebenenfalls Alkylenoxid sind, die durch die allgemeine Formel I

$$H_O R_3 (NR_1 R_2)_p$$

$R_1, R_2$:   $CHR=CH-CH_2$, H      für o=0 ist p=2

       R:    H, $CH_3$      für o=1 ist p=1

$R_3$:  $R_a-C(H)_c(R_6)-C_2$

       $R_a$:    monovalent für c=0: H
              divalent für c=1: $CH_2$,
                     $OCH_2$,
                     $C_{1-6}-O-(CHR-CHR-O)_d$,      d=1-45
                     $C_{1-6}-CO-O-(CHR-CHR-O)_d$

       $R_6$:   $R_7H$

          $R_7$:    O, $O(CHR-CHR-O)_n$      n: 1 bis 45

beschrieben werden können, mit der Maßgabe, daß mindestens 2 Reste $R_1$, $R_2$ CHR = CH - $CH_2$ bedeuten.

3. Polymerisat nach Anspruch 1, dadurch gekennzeichnet, daß die Vernetzer Umsetzungsprodukte aus (Meth)allylaminen mit Di-, Tri- oder Tetraglycidylverbindungen und gegebenenfalls Alkylenoxid sind, die durch die allgemeine Formel II

$$R_8 (NR_1 R_2)_2$$

$R_1, R_2$:   $CHR=CH-CH_2$, H

          R:    H, $CH_3$

$R_8$:  $CH_2-CH(R_6)-CH_2-O-R_4-CH_2-CH(R_6)-CH_2$

       $R_4$:    $(CHR-CHR-O)_m$,      m=1 bis 45
              $C_1-C_6-O$,
              $R_5(O-R_3-NR_1R_2)_r-O$

              r=0: bei Verwendung von Diglycidverbindungen
              r=1: bei Verwendung von Triglycidverbindungen
              r=2: bei Verwendung von Tetraglycidverbindungen

          $R_5$:    Alkylenrest aus einem Polyol, gegebenenfalls mit einer oder zwei OH-Funktionen, die optional mit bis zu 45 Mol Alkylenoxid

          umgesetzt sein können.

$R_3$:    $CH_2-CH(R_6)-CH_2$

18

$R_6$:    $R_7H$

$R_7$:    O, O(CHR-CHR-O)$_n$      n: 1 bis 45

beschrieben werden können, mit der Maßgabe, daß mindestens 2 Reste $R_1$, $R_2$ CHR = CH - CH$_2$ bedeuten.

**4.** Polymerisat nach Anspruch 1, dadurch gekennzeichnet, daß die Vernetzer Umsetzungsprodukte aus (Meth)allylaminen bzw. gesättigten primären Aminen mit (Meth)acrylglycidestem und/oder (Meth)allylglycidethem und gegebenenfalls Alkylenoxid sind, die durch die allgemeine Formel III

$$(R_9\text{-}R_3)_a N(B)_c (R_1)_j (R_2)_k$$

B:       $C_1$-$C_6$-Alkyl    <u>für c=0 gilt;</u>
$R_9$:      CHR=CH-CH$_2$-O,     für a=1 sind j=1 und k=1
            CH$_2$=CR-CO-O     für a=2 sind j=1 und k=0
$R_1$,$R_2$:    CHR=CH-CH$_2$, H     für a=3 sind j und k =0

R:    H, CH$_3$

$R_3$:   CH$_2$-CH($R_6$)-CH$_2$    <u>Für c=1 gilt:</u>

$R_6$:    $R_7H$     für a=1 sind j=1 und k=0

$R_7$:    O, O(CHR-CHR-O)$_n$      a=2 sind j und k=0
       n: 1 bis 45

beschrieben werden können.

**5.** Polymerisat nach Anspruch 1, dadurch gekennzeichnet, daß die Vernetzer Umsetzungsprodukte aus Di- oder Polyaminen mit (Meth)allylglycidethern und/oder (Meth)acrylglycidestem und gegebenenfalls Alkylenoxid sind, die durch die allgemeine Formel IV

$$R_{11}[N(A)_b\text{-}(R_3\text{-}R_9)_p]_z$$

für b=1 ist p=1

für b=0 ist p=2

z=2, 3, 4

A:       H, $C_1$-$C_6$-Alkyl
$R_3$:      CH$_2$-CH($R_6$)-CH$_2$

$R_6$:    $R_7H$

$R_7$:    O, O(CHR-CHR-O)$_n$      n: 1 bis 45

R:    H, CH$_3$

$R_9$:   CHR=CH-CH$_2$-O,
       CH$_2$=CR-CO-O
R11: di-, tri- oder tetravalenter Alkylenrest aus der Aminkomponente (gemischt oder einzeln aliphatisch, cyloaliphatisch, aromatisch, heterocyclisch)

beschrieben werden können.

**6.** Polymerisat nach einem der Ansprüche 1 bis 5, dadurch gekennzeichnet, daß die Vernetzer mindestens eine (Meth)allylgruppe enthalten.

**7.** Polymerisat nach Anspruch 1, dadurch gekennzeichnet, daß es unter Verwendung von 1,3-Bis-(diallylamino)-propanol-2 hergestellt wurde.

**8.** Polymerisat nach Anspruch 1, dadurch gekennzeichnet, daß es unter Verwendung von (Poly)ethylenglykol-di(N, N-diallyl-3-amino-2-hydroxy-prop-1-yl)ether hergestellt wurde

**9.** Polymerisat nach Anspruch 1, dadurch gekennzeichnet, daß es unter Verwendung von 1-Allyloxy-2-hydroxy-3-N, N-diallylamino-propan hergestellt wurde.

**10.** Polymerisat nach einem der Ansprüche 1 bis 9, dadurch gekennzeichnet, daß die Vernetzer zusätzlich alkoxyliert sind.

**11.** Polymerisat nach Anspruch 10, dadurch gekennzeichnet, daß die Vernetzer mit bis zu 45 Mol Alkylenoxid, vorzugsweise mit bis zu 20 Mol Alkylenoxid und besonders bevorzugt mit bis zu 12 Mol Alkylenoxid pro Mol Hydroxylgruppe umgesetzt sind.

**12.** Polymerisat nach einem der Ansprüche 1 bis 11, dadurch gekennzeichnet, daß die Vernetzer bzw. ihre Mischungen mit 0,01 - 3,0 Gew. %, vorzugsweise 0,05 - 1,5 Gew. % und besonders bevorzugt mit 0,1 - 1,0 Gew.% bezogen auf die Monomeren eingesetzt werden.

**13.** Polymerisat nach einem der Ansprüche 1 - 12, dadurch gekennzeichnet, daß weitere Covernetzer mit 0 - 1 Gew. %, bevorzugt mit 0,01 - 0,8 und besonders bevorzugt mit 0,05 - 0,4 Gew. % bezogen auf die Monomeren mitverwendet werden.

**14.** Polymerisat nach Anspruch 13, dadurch gekennzeichnet, daß als Covemetzer Triallylamin, N,N-Methylenbisacrylamid, Bis(acrylamido)essigsäure, Allyl(meth)acrylat, Trimethylolpropantriacrylat, ethoxyliertes Trimethylolpropantriacrylat, Polyglykoldiacrylat mitverwendet werden.

**15.** Polymerisat nach einem der Ansprüche 1 bis 14, dadurch gekennzeichnet, daß die ungesättigten Säuregruppen tragenden Monomeren aus der Gruppe Acrylsäure, Methacrylsäure, Vinylessigsäure, Vinylsulfonsäure, Methallylsulfonsäure und 2-Acrylamido-2-methylpropansulfonsäure ausgewählt sind.

**16.** Polymerisat nach einem der Ansprüche 1 bis 15, dadurch gekennzeichnet, daß es, bezogen auf die sauren Monomere, 0 bis 40 Gew. % weitere Comonomere aus der Gruppe (Meth)acrylamid, (Meth)acrylnitril, Vinylpyrrolidon, Hydroxyethylacrylat und Vinylacetamid einpolymerisiert enthält.

**17.** Polymerisat nach einem der Ansprüche 1 bis 16, dadurch gekennzeichnet, daß es, bezogen auf die Summe der vorhandenen Monomere, bis zu 30 Gew.% wasserlösliches Polymer als Pfropfgrundlage, bevorzugt Polysaccharide und/oder Polyvinylalkohol enthält.

**18.** Polymerisat nach einem der Ansprüche 1 bis 17, dadurch gekennzeichnet, daß es an der Oberfläche mit einem Nachvemetzer vernetzt wurde und diese Nachvernetzung gegebenfalls mehrfach wiederholt wurde.

**19.** Polymerisat nach Anspruch 18, dadurch gekennzeichnet, daß es an der Oberfläche mit einem Nachvernetzer aus der Gruppe der Polyole, Polyepoxide, Polyamine oder Alkylencarbonate vernetzt wurde.

**20.** Polymerisat nach Anspruch 18 und 19, dadurch gekennzeichnet, daß es eine Retention von mindestens 30 g/g, eine Flüssigkeitaufnahme unter Druck (49 g/cm$^2$) von mindestens 20 g/g und eine Permeabilität GLP von mindestens $10 \cdot 10^{-7}$ cm$^3$ sec/g hat sowie einen Quelldruck (20 min) von mindestens 600 g aufweist.

**21.** Polymerisat nach Anspruch 18 bis 20, dadurch gekennzeichnet, daß es eine Permeabilität GLP von mindestens $15 \cdot 10^{-7}$ cm$^3$ sec/g und bevorzugt von mindestens $25 \cdot 10^{-7}$ cm$^3$ sec/g hat.

**22.** Polymerisat nach einem der Ansprüche 18 bis 21, dadurch gekennzeichnet, daß es einen Quelldruck (20 min) von mindestens 900 g, bevorzugt von mindestens 1000 g aufweist.

**23.** Polymerisat nach einem der Ansprüche 18 bis 22, dadurch gekennzeichnet, daß es eine Flüssigkeitaufnahme unter Druck (49 g/cm$^2$) von mindestens 23 g/g aufweist.

**24.** Verfahren zur Herstellung eines wäßrige Flüssigkeiten absorbierenden, vernetzten Polymerisates nach einem der Ansprüche 1 bis 17, dadurch gekennzeichnet, daß eine wäßrige Lösung aus ungesättigten, Säuregruppen tragenden, teilneutralisierten Monomeren unter Verwendung von Vernetzern aus mehrfach ungesättigten Aminoalkoholen und gegebenenfalls weiteren Covernetzern unter Zusatz von Radikalbildnern nach dem Verfahren einer Lösungs- oder Suspensionspolymerisation zu einem Hydrogel polymerisiert, zerkleinert, getrocknet, gemahlen und gesiebt wird.

**25.** Verfahren nach Anspruch 24, dadurch gekennzeichnet, daß die Polymerisate mit wenigstens einem Oberflächenvernetzer behandelt werden und eine Oberflächenvernetzung bei erhöhter Temperatur durchgeführt wird.

**26.** Verfahren nach Anspruch 25, dadurch gekennzeichnet, daß die Oberflächenbehandlung und Vernetzung mehrmals durchgeführt wird.

**27.** Verwendung der Polymerisate gemäß der Ansprüche 1 bis 23 als Absorptionsmittel für Wasser und wäßrige Flüssigkeiten.

**28.** Verwendung von Polymerisaten nach den Ansprüchen 1 bis 23 in Konstruktionen zur Aufnahme von Körperflüssigkeiten.

**29.** Verwendung von Folymerisaten nach den Ansprüchen 1 bis 23 als Wasser oder wäßrige Flüssigkeiten absorbierende Komponente in strom -oder lichtleitenden Kabeln, als Komponente in Verpackungsmaterialien, als Bodenverbesserungsmittel und bei der Pflanzenaufzucht.

**30.** Verwendung von Polymerisaten nach den Ansprüchen 1 bis 23 in Wasser oder wäßrige Flüssigkeiten absorbierenden, geschäumten oder nicht geschäumten Flächengebilden.

**31.** Verwendung von Polymerisaten nach den Ansprüchen 1 bis 23 als Trägersubstanz für Düngemittel oder andere Wirkstoffe, die wieder über einen längeren Zeitraum verzögert an die Umgebung abgegeben werden.

**32.** Vernetzer der allgemeinen Formel I

$$H_o R_3 (NR_1 R_2)_p$$

$R_1, R_2$:  CHR=CH-CH$_2$, H      für o=0 ist p=2

R:   H, CH$_3$      für o=1 ist p=1

$R_3$:  $R_a$-C(H)$_c$(R$_6$)-CH$_2$

$R_a$:   monovalent für c=0: H
divalent      für c=1: CH$_2$,
OCH$_2$,
C$_{1-6}$-O-(CHR-CHR-O)$_d$,      d=1-45
C$_{1-6}$-CO-O-(CHR-CHR-O)$_d$

$R_6$:   R$_7$H

$R_7$:   O, O(CHR-CHR-O)$_n$      n: 1 bis 45

mit der Maßgabe, daß mindestens 2 Reste $R_1$, $R_2$ CHR = CH - CH$_2$ bedeuten.

**33.** Vernetzer der allgemeinen Formel II

$$R_8(NR_1R_2)_2$$

$R_1, R_2$:     $CHR=CH-CH_2$, H

       R:     H, $CH_3$

$R_8$:   $CH_2-CH(R_6)-CH_2-O-F_4-CH_2-CH(R_6)-CH_2$

       $R_4$:     $(CHR-CHR-O)_m$,       m=1 bis 45
              $C_1-C_6-O$,
              $R_5(O-R_3-NR_1R_2)_r-O$

              r=0: bei Verwendung von Diglycidverbindungen
              r=1: bei Verwendung von Triglycidverbindungen
              r=2: bei Verwendung von Tetraglycidverbindungen

       R5:     Alkylenrest aus einem Polyol, gegebenenfalls mit einer oder zwei OH-Funktionen, die optional mit bis zu 45 Mol Alkylenoxid umgesetzt sein können.

       $R_3$:     $CH_2-CH(R_6)-CH_2$

           $R_6$:     $R_7$H

              $R_7$:     O, $O(CHR-CHR-O)_n$       n: 1 bis 45

mit der Maßgabe, daß mindestens 2 Reste $R_1$, $R_2$ CHR = CH - $CH_2$ bedeuten.

**34.** Vernetzer der allgemeinen Formel III

$$(R_9-R_3)_a N(B)_c (R_1)_j (R_2)_k$$

B:        $C_1-C_6$-Alkyl     <u>für c=0 gilt:</u>
$R_9$:        $CHR=CH-CH_2-O$,       für a=1 sind j=1 und k=1
            $CH_2=CR-CO-O$       für a=2 sind j=1 und k=0
$R_1, R_2$:        $CHR=CH-CH_2$, H       für a=3 sind j und k =0

              R:     H, $CH_3$

$R_3$:   $CH_2-CH(R_6)-CH_2$     <u>Für c=1 gilt:</u>

       $R_6$:     $R_7$H       für a=1 sind j=1 und k=0

           $R_7$:     O, $O(CHR-CHR-O)_n$       a=2 sind j und k=0

           n:     1 bis 45

**35.** Vernetzer der allgemeinen Formel IV

$$R_{11} [N(A)_b - (R_3-R_9)_p]_z$$

       für b=1 ist p=1

       für b=0 ist p=2

z=2, 3, 4

A: H, $C_1$-$C_6$-Alkyl
$R_3$: $CH_2$-$CH(R_6)$-$CH_2$

$R_6$: $R_7$H

$R_7$: O, $O(CHR\text{-}CHR\text{-}O)_n$    n: 1 bis 45

R: H, $CH_3$

$R_9$: $CHR\text{=}CH\text{-}CH_2\text{-}O,$
$CH_2\text{=}CR\text{-}CO\text{-}O$
R11: di-, tri- oder tetravalenter Alkylenrest aus der Aminkomponente (gemischt oder einzeln aliphatisch, cyloaliphatisch, aromatisch, heterocyclisch)

**36.** Vernetzer gemäß der Ansprüche 1 bis 6, 10, 11 und 32 bis 35, dadurch gekennzeichnet, daß die Aminfunktionen quaterniert sind.

**37.** Verwendung der Verbindungen gemäß der Ansprüche 32 bis 35 zur Vernetzung und/oder Verzweigung von Polymeren in Lösung oder Substanz.

**Claims**

**1.** A cross-linked polymer absorbing aqueous liquids, built-up of partially neutralized, monoethylenically unsaturated, acid groups-containing monomers, optionally further monomers copolymerizable with these, as well as optional polymers suitable as a graft basis, characterized in that it can be produced by using cross-linking agents of polyunsaturated amino alcohols.

**2.** The polymer according to claim 1 characterized in that the cross-linking agents are reaction products of (meth) allylamines with monoglycidyl compounds and optional alkylene oxide, which may be described by the general formula I

$$H_oR_3(NR_1R_2)_p$$

$R_1,R_2$: $CHR\text{=}CH\text{-}CH_2$,H    for o=0 is p=2

R: H, $CH_3$    for o = 1 is p =1

$R_3$: $R_a\text{-}C(H)_c(R_6)\text{-}CH_2$

$R_a$: monovalent    for c = 0: H
divalent for c =1: $CH_2$,
$OCH_2$,
$C_{1\text{-}6}\text{-}O\text{-}(CHR\text{-}CHR\text{-}O)_d$, d = 1-45
$C_{1\text{-}6}\text{-}CO\text{-}O\text{-}(CHR\text{-}CHR\text{-}O)_d$

$R_6$: $R_7$H

$R_7$: O, $O(CHR\text{-}CHR\text{-}O)_n$    n: 1 to 45,

with the proviso that at least 2 residues $R_1,R_2$ mean $CHR\text{=}CH\text{-}CH_2$.

**3.** The polymer according to claim 1, characterized in that the cross-linking agents are reaction products of (meth) allylamines with di-, tri-, or tetraglycidyl compounds and optional alkylene oxide, which can be described by the general formula II

$$R_8(NR_1R_2)_2$$

$R_1, R_2$:   $CHR=CH-CH_2, H$

       R:   $H, CH_3$

$R_8$:  $CH_2-CH(R_6)-CH_2-O-R_4-CH_2-CH(R_6)-CH_2$

      $R_4$:   $(CHR-CHR-O)_m$,     $m = 1$ to $45$
            $C_1-C_6-O$,
            $R_5(O-R_3-NR_1R_2)_r-O$

            $r=0$: if diglycide compounds are used
            $r = 1$: if triglycide compounds are used
            $r=2$: if tetraglycide compounds are used

      $R_5$:   alkylene residue of a polyol, optionally with one or two OH-functions which may optionally be reacted with up to 45 moles of alkylene oxide

$R_3$:   $CH_2-CH(R_6)-CH_2$

      $R_6$:   $R_7H$

         $R_7$:   $O, O(CHR-CHR-O)_n$    $n$: $1$ to $45$,

with the proviso that at least 2 residues $R_1, R_2$ mean $CHR = CH-CH_2$.

4.   The polymer according to claim 1 characterized in that the cross-linking agents are reaction products of (meth) allylamines or saturated primary amines with (meth)acrylglycide esters and/or (meth)allyl glycidyl ethers and optional alkylene oxide, which can be described by the general formula III

$$(R_9-R_3)_a N(B)_c (R_1)_j (R_2)_k$$

B:       $C_1-C_6$-alkyl    <u>for c=0 the following is valid:</u>
$R_9$:      $CHR=CH-CH_2-O$,    for a=1 is j=1 and k = 1
           $CH_2=CR-CO-O$    for a=2 is j=1 and k=0
$R_1, R_2$:  $CHR=CH-CH_2, H$    for a=3 is j and k =0

      R:   $H, CH_3$

$R_3$:  $CH_2-CH(R_6)-CH_2$    <u>for c = 1 the following is valid:</u>

      $R_6$:   $R_7H$    for a=1 is j=1 and k=0

         $R_7$:   $O, O(CHR-CHR-O)_n$    a=2 is j and k=0
             $n$: $1$ to $45$

5.   The polymer according to claim 1 characterized in that the cross-linking agents are reaction products of di- or polyamines with (meth)allyl glycidyl ethers and/or (meth)acrylglycide esters and optional alkylene oxide, which can be described by the general formula IV

$$R_{11}[N(A)_b-(R_3-R_9)_p]_z$$

    for b=1 is p=1

for b=0 is p=2

z=2,3,4

A: $H, C_1-C_6$-alkyl
$R_3$: $CH_2-CH(R_6)-CH_2$

$R_6$: $R_7H$

$R_7$: $O, O(CHR-CHR-O)_n$    n: 1 to 45

R: $H, CH_3$

$R_9$: $CHR = CH-CH_2-O$,
$CH_2 = CR-CO-O$
R11 : di-, tri- or tetravalent alkylene residue of the amine component (mixed or individually aliphatic, cycloaliphatic, aromatic, heterocyclic).

6. The polymer according to any one of claims 1 to 5 characterized in that the cross-linking agents comprise at least one (meth) allyl group.

7. The polymer according to claim 1 characterized in that it has been manufactured by using 1,3-bis-(diallylamino)-2-propanol.

8. The polymer according to claim 1 characterized in that it has been manufactured by using polyethylene glycol di (N,N-diallyl-3-amino-2-hydroxy-prop-1-yl)ether.

9. The polymer according to claim 1 characterized in that it has been manufactured by using 1-allyloxy-2-hydroxy-3-N,N-diallylaminopropane.

10. The polymer according to any one of claims 1 to 9 characterized in that the cross-linking agents are additionally alkoxylated.

11. The polymer according to claim 10 characterized in that the cross-linking agents are reacted with up to 45 moles of alkylene oxide, preferably up to 20 moles of alkylene oxide, and most preferably up to 12 moles of alkylene oxide per mole of hydroxyl group.

12. The polymer according to any one of claims 1 to 11 characterized in that the cross-linking agents or their mixtures are used in amounts of 0.01 - 3.0%-wt., preferably 0.05 - 1.5%-wt., and most preferably 0.1 - 1.0%-wt., relative to the monomers.

13. The polymer according to any one of claims 1 - 12 characterized in that further cocross-linkers are used in amounts of 0 - 1 %-wt., preferably 0.01 - 0.8, and most preferably 0.05 - 0.4%-wt., relative to the monomers.

14. The polymer according to claim 13 characterized in that tri-allylamine, N,N-methylenebisacrylamide, bis(acrylamido) acetic acid, allyl (meth)acrylate, trimethylolpropane triacrylate, ethoxylated trimethylolpropane triacrylate, polyglycol diacrylate are used as co-cross-linkers.

15. The polymer according to any one of claims 1 to 14 characterized in that the unsaturated, acid groups-containing monomers are selected from the group consisting of acrylic acid, methacrylic acid, vinyl acetic acid, vinyl sulfonic acid, methallyl sulfonic acid, and 2-acrylamido-2-methylpropane sulfonic acid.

16. The polymer according to any one of claims 1 to 15 characterized in that it comprises incorporated by polymerization 0 to 40%-wt., relative to the acid monomers, of further comonomers from the group (meth)acrylamide, (meth) acrylonitrile, vinyl pyrrolidone, hydroxyethyl acrylate, and vinyl acetamide.

17. The polymer according to any one of claims 1 to 16 characterized in that it comprises up to 30%-wt., relative to the sum of existing monomers, of a water-soluble polymer as a graft basis, preferably polysaccharides and/or polyvinyl alcohol.

18. The polymer according to any one of claims 1 to 17 characterized in that it has been cross-linked at the surface with a secondary cross-linking agent, and that this re-cross-linkage has optionally been repeated several times.

19. The polymer according to claim 18 characterized in that it has been cross-linked at the surface with a secondary cross-linking agent of the group polyols, polyepoxides, polyamines, or alkylene carbonates.

20. The polymer according to claim 18 and 19 characterized in that it has a retention of at least 30 g/g, a liquid absorption under pressure (49 g/cm$^2$) of at least 20 g/g, and a permeability GLP of at least $10 \cdot 10^{-7}$ cm$^3$ sec./g, as well as a swelling pressure (20 min.) of at least 600 g.

21. The polymer according to claim 18 to 20 characterized in that it has a permeability GLP of at least $15 \cdot 10^{-7}$ cm$^3$ sec./g, and preferably at least $25 \cdot 10^{-7}$ cm$^3$ sec./g.

22. The polymer according to any one of claims 18 to 21 characterized in that it has a swelling pressure (20 min.) of at least 900 g, preferably at least 1,000 g.

23. The polymer according to any one of claims 18 to 22 characterized in that has a liquid absorption under pressure (49 g/cm$^2$) of at least 23 g/g.

24. A process for the production of a cross-linked polymer absorbing aqueous liquids according to any one of claims 1 to 17, characterized in that an aqueous solution of unsaturated, acid groups-containing, partially neutralized monomers is polymerized into a hydrogel according to the method of a solution or suspension polymerization, with using cross-linking agents of polyunsaturated amino alcohols and optional further cocross-linkers under addition of radical formers, is comminuted, dried, ground, and screened out.

25. The process according to claim 24 characterized in that the polymers are treated with at least one surface cross-linking agent, and that a surface cross-linkage is carried out at elevated temperatures,

26. The process according to claim 25 characterized in that the surface treatment and the cross-linkage are carried out several times.

27. The use of the polymers according to claims 1 to 23 as absorbents for water and aqueous liquids.

28. The use of the polymers according to claims 1 to 23 in constructions for the absorption of body fluids.

29. The use of the polymers according to claims 1 to 23 as a component which absorbs water or aqueous liquids in current-conducting or light-transmitting cables, as a component in packaging materials, as soil conditioner, and in plant cultivation.

30. The use of the polymers according to claims 1 to 23 in foamed or unfoamed sheet materials absorbing water or aqueous liquids.

31. The use of the polymers according to claims 1 to 23 as a carrier substance for fertilizers or other active substances which are released to the environment in a retarded manner over a longer period of time.

32. A cross-linking agent according to the general formula I

$$H_o R_3 (NR_1 R_2)_p$$

$R_1, R_2$:    $CHR=CH-CH_2, H$     for o=0 is p=2

           R:   H, $CH_3$     for o=1 is p=1

$R_3$:  $R_a - C(H)_c (R_6) - CH_2$

$R_a$:    monovalent     for c=0: H

       divalent for c=1:      $CH_2$,

                  $OCH_2$,

                  $C_{1-6}$-O-(CHR-CHR-O)$_d$, d=1-45

                  $C_{1-6}$-CO-O-(CHR-CHR-O)$_d$

$R_6$:    $R_7H$

$R_7$:    O, O(CHR-CHR-O)$_n$      n: 1 to 45,

with the proviso that at least 2 residues $R_1,R_2$ mean $CHR=CH-CH_2$,

**33.** A cross-linking agent according to the general formula II

$$R_8(NR_1R_2)_2$$

$R_1,R_2$:    $CHR=CH-CH_2$,H

        R:    H, $CH_3$

$R_8$:   $CH_2$-CH($R_6$)-$CH_2$-O-$R_4$-$CH_2$-CH($R_6$)-$CH_2$

       $R_4$:    (CHR-CHR-O)$_m$,      m = 1 to 45

             $C_1$-$C_6$-O,

             $R_5$(O-$R_3$-$NR_1R_2$)$_r$-O

                  r=0: if diglycide compounds are used

                  r = 1: if triglycide compounds are used

                  r=2: if tetraglycide compounds are used

         $R_5$:    alkylene residue of a polyol, optionally with one or two OH-functions which may optionally be reacted with up to 45 moles of alkylene oxide

         $R_3$:    $CH_2$-CH($R_6$)-$CH_2$

              $R_6$:    $R_7H$

                 $R_7$:    O, O(CHR-CHR-O)$_n$ n: 1 to 45,

with the proviso that at least 2 residues $R_1,R_2$ mean $CHR=CH-CH_2$.

**34.** A cross-linking agent according to the general formula III

$$(R_9-R_3)_a N(B)_c (R_1)_j (R_2)_k$$

B:        $C_1$-$C_6$-alkyl      <u>for c = 0 the following is valid:</u>

$R_9$:       $CHR=CH-CH_2$-O,       for a=1 is j=1 and k=1

           $CH_2$=CR-CO-O     for a=2 is j=1 and k=0

$R_1,R_2$:    $CHR=CH-CH_2$,H     for a=3 is j and k = 0

        R:    H, $CH_3$

        $R_3$:    $CH_2$-CH($R_6$)-$CH_2$       <u>for c = 1 the following is valid:</u>

           $R_6$:    $R_7H$     for a=1 is j=1 and k=0

           $R_7$:    O, O(CHR-CHR-O)$_n$     a=2 is j and k=0

               n: 1 to 45

**35.** A cross-linking agent according to the general formula IV

$$R_{11}[N(A)_b\text{-}(R_3\text{-}R_9)_p]_z$$

for b=1 is p=1

for b=0 is p=2

z=2,3,4

A:      H,$C_1$-$C_6$-alkyl
$R_3$:     $CH_2$-$CH(R_6)$-$CH_2$

  $R_6$:   $R_7$H

    $R_7$:   O, O(CHR-CHR-O)$_n$ n: 1 to 45

      R:   H, $CH_3$

$R_9$:  CHR = CH-$CH_2$-O,
       $CH_2$ = CR-CO-O
R11 :di-, tri- or tetravalent alkylene residue of the amine component (mixed or individually aliphatic, cycloaliphatic, aromatic, heterocyclic).

**36.** The cross-linking agent according to claims 1 to 6, 10, 11, and 32 to 35 characterized in that the amine functions are quaternized.

**37.** The use of the compounds according to claims 32 to 35 for cross-linking and/or branching polymers in solution or substance.

**Revendications**

**1.** Polymère réticulé absorbant des liquides aqueux, constitué de monomères portant des groupes acide monoéthyléniquement insaturés partiellement neutralisés, éventuellement d'autres monomères copolymérisables avec eux, ainsi qu'éventuellement de polymères appropriés comme base de greffage, caractérisé en ce qu'il peut être préparé par l'utilisation d'agents de réticulation à base d'aminoalcools plusieurs fois insaturés.

**2.** Polymère suivant la revendication 1, caractérisé en ce que les agents de réticulation sont des produits de réaction de (méth)-allylamines avec des composés monoglycidyliques et éventuellement un oxyde d'alkylène, produits qui peuvent être décrits par la formule générale I:

$$H_oR_3(NR_1R_2)_p$$

$R_1$, $R_2$:     CHR=CH-$CH_2$, H       pour o = 0, p = 2

    R:   H, $CH_3$       pour o = 1, p = 1

$R_3$ :  $R_a$-$C(H)_c(R_6)$-$CH_2$

    $R_a$ :    monovalent pour c = 0 : H
          divalent pour c = 1 : $CH_2$
                $OCH_2$
          $C_{1-6}$-O-(CHR-CHR-O)$_d$       d = 1-45
          $C_{1-6}$-CO-O(CHR-CHR-O)$_d$

$R^6$ :   $R_7H$

$R_7$:   O, O(CHR-CHR-O)$_n$     n : 1 à 45

à la condition qu'au moins deux radicaux $R_1$, $R_2$ représentent CHR=CH-CH$_2$.

**3.** Polymère suivant la revendication 1, caractérisé en ce que les agents de réticulation sont des produits de réaction de (méth)allylamines avec des composés di-, tri- ou tétraglycidyliques et éventuellement un oxyde d'alkylène, produits qui peuvent être décrits par la formule générale II :

$$R_8(NR_1R_2)_2$$

$R_1$, $R_2$:   CHR=CH-CH$_2$, H

R:   H, CH$_3$

$R_8$:  CH$_2$-CH(R$_6$)-CH$_2$-O-R$_4$-CH$_2$-CH(R$_6$)-CH$_2$

$R_4$ :   (CHR-CHR-O)$_m$     m = 1 à 45
C$_1$-C$_6$-O
R$_5$(O-R$_3$-NR$_1$R$_2$)$_r$-O

r = 0 : lors de l'utilisation de composés diglycidiques
r = 1 : lors de l'utilisation de composés triglycidiques
r = 2 : lors de l'utilisation de composés tétraglycidiques,

$R_5$:   radical alkylène à base d'un polyol, éventuellement comportant une ou deux fonctions OH, qui peuvent éventuellement être mises à réagir avec jusqu'à 45 moles d'oxyde d'alkylène,

$R_3$ :   CH$_2$-CH(R$_6$)-CH$_2$

$R_6$ :   $R_7H$

$R_7$:   O, O(CHR-CHR-O)$_n$     n = 1 à 45

à la condition qu'au moins deux radicaux $R_1$, $R_2$ représentent CHR=CH-CH$_2$.

**4.** Polymère suivant la revendication 1, caractérisé en ce que les agents de réticulation sont des produits de réaction de (méth)allylamines ou respectivement d'amines primaires saturées avec des esters (méth)acrylglycidiques et/ ou des éthers (méth)allyglycidiques et éventuellement un oxyde d'alkylène, produits qui peuvent être décrits par la formule générale III :

$$(R_9\text{-}R_3)_a N(B)_c (R_1)_j (R_2)_k$$

B :     C$_1$-C$_6$-alkyle     pour c = 0, on a :
$R_9$ :     CHR=CH-CH$_2$-O     pour a = 1, j = 1 et k = 1
CH$_2$=CR-CO-O     pour a = 2, j = 1 et k = 0
$R_1$, $R_2$ :   CHR=CH-CH$_2$, H     pour a = 3, j et k = 0

R:   H, CH$_3$

$R_3$ :  CH$_2$-CH(R$_6$)-CH$_2$     pour c = 1, on a :

$R_6$:   $R_7H$     pour a = 1, j = 1 et k = 0
$R_7$ :   O, O(CHR-CHR-O)$_n$     pour a = 2, j et k = 0
n : 1 à 45.

**5.** Polymère suivant la revendication 1, caractérisé en ce que les agents de réticulation sont des produits de réaction de diamines ou polyamines avec des éthers (méth)allyglycidiques et/ou des esters (méth)acrylglycidiques et éventuellement un oxyde d'alkylène, produits qui peuvent être décrits par la formule générale IV :

$$R_{11}[N(A)_b\text{-}(R_3\text{-}R_9)_p]_z$$

pour b = 1, p = 1

pour b = 0, p = 2

z = 2, 3, 4

A : H, $C_1$-$C_6$-alkyle

$R_3$ : $CH_2$-$CH(R_6)$-$CH_2$

    $R_6$ : $R_7$H

        $R_7$ : O, $O(CHR\text{-}CHR\text{-}O)_n$     n : 1 à 45

            R: H, $CH_3$

$R_9$ : $CHR=CH\text{-}CH_2\text{-}O$,

       $CH_2=CR\text{-}CO\text{-}O$

R11 : radical alkylène di-, tri- ou tétravalent du composant aminé (en mélange ou isolément aliphatique, cycloaliphatique, aromatique, hétérocyclique).

**6.** Polymère suivant l'une des revendications 1 à 5, caractérisé en ce que les agents de réticulation comprennent au moins un groupe (méth)allyle.

**7.** Polymère suivant la revendication 1, caractérisé en qu'il est préparé par utilisation de 1,3-bis-(diallylamino)-propanol-2.

**8.** Polymère suivant la revendication 1, caractérisé en ce qu'il est préparé par utilisation d'éther (poly)éthylèneglycol-di(N,N-diallyl-3-amino-2-hydroxy-prop-1-ylique).

**9.** Polymère suivant la revendication 1, caractérisé en ce qu'il est préparé par utilisation de 1-allyloxy-2-hydroxy-3-N, N-diallyl-amino-propane.

**10.** Polymère suivant l'une des revendications 1 à 9, caractérisé en ce que les agents de réticulation sont en supplément alcoxylés.

**11.** Polymère suivant la revendication 10, caractérisé en ce que les agents de réticulation sont mis à réagir avec jusqu'à 45 moles d'oxyde d'alkylène, de préférence avec jusqu'à 20 moles d'oxyde d'alkylène, et particulièrement préférentiellement avec jusqu'à 12 moles d'oxyde d'alkylène, par mole de groupe hydroxyle.

**12.** Polymère suivant l'une des revendications 1 à 11, caractérisé en ce que les agents de réticulation ou leurs mélanges sont mis en oeuvre à 0,01-3,0% en poids, de préférence 0,05-1,5% en poids, et particulièrement préférentiellement à 0,1-1,0% en poids, par rapport aux monomères.

**13.** Polymère suivant l'une des revendications 1 à 12, caractérisé en ce que d'autres coagents de réticulation sont conjointement utilisés à 0-1% en poids, de préférence à 0,01-0,8% en poids et particulièrement préférentiellement à 0,05-0,4% en poids, par rapport aux monomères.

**14.** Polymère suivant la revendication 13, caractérisé en ce que, comme coagents de réticulation, on utilise conjointement de la triallylamine, du N,N-méthylènebisacrylamide, de l'acide bis(acrylamido)-acétique, du (méth)acrylate d'allyle, de l'acrylate de triméthylolpropane, du triacrylate de triméthylolpropane éthoxylé, du diacrylate de polyglycol.

**15.** Polymère suivant l'une des revendications 1 à 14, caractérisé en ce que les monomères portant des groupes acide insaturés sont choisis parmi le groupe de l'acide acrylique, de l'acide méthacrylique, de l'acide vinylacétique, de l'acide vinylsulfonique, de l'acide méthallylsulfonique et de l'acide 2-acrylamido-2-méthylpropane-sulfonique.

**16.** Polymère suivant l'une des revendications 1 à 15, caractérisé en ce que, par rapport aux monomères acides, il contient 0 à 40% en poids d'autres comonomères du groupe du (méth)acrylamide, du (méth)acrylonitrile, de la vinylpyrrolidone, de l'acrylate d'hydroxyéthyle et du vinylacétamide.

**17.** Polymère suivant l'une des revendications 1 à 16, caractérisé en ce que, par rapport à la somme des monomères présents, il contient jusqu'à 30% en poids de polymère soluble dans l'eau comme base de greffage, de préférence des poysaccharides et/ou de l'alcool polyvinylique.

**18.** Polymère suivant l'une des revendications 1 à 17, caractérisé en ce qu'il est réticulé à la surface avec un agent de postréticulation et en ce que cette postréticulation est éventuellement répétée à plusieurs reprises.

**19.** Polymère suivant la revendication 18, caractérisé en ce qu'il est réticulé à la surface avec un agent de postréticulation du groupe des polyols, des polyépoxydes, des polyamines ou des carbonates d'alkylène.

**20.** Polymère suivant l'une des revendications 18 et 19, caractérisé en ce qu'il présente une rétention d'au moins 30 g/g, une absorption de liquide sous pression (49 g/cm$^2$) d'au moins 20 g/g et une perméabilité GLP d'au moins $10 \cdot 10^{-7}$ cm$^3$ s/g ainsi qu'une pression de gonflement (20 minutes) d'au moins 600 g.

**21.** Polymère suivant l'une des revendications 18 à 20, caractérisé en ce qu'il a une perméabilité GLP d'au moins $15 \cdot 10^{-7}$ cm$^3$ s/g et de préférence d'au moins $25 \cdot 10^{-7}$ cm$^3$ s/g.

**22.** Polymère suivant l'une des revendications 18 à 21, caractérisé en ce qu'il présente une pression de gonflement (20 minutes) d'au moins 900 g, de préférence d'au moins 1000 g.

**23.** Polymère suivant l'une des revendications 18 à 22, caractérisé en ce qu'il présente une absorption de liquide sous pression (49 g/cm$^2$) d'au moins 23 g/g.

**24.** Procédé de préparation d'un polymère réticulé, absorbant des liquides aqueux, suivant l'une des revendications 1 à 17, caractérisé en ce qu'une solution aqueuse à base de monomères partiellement neutralisés, portant des groupes acide, insaturés, est polymérisée avec utilisation d'agents de réticulation à base d'aminoalcools plusieurs fois insaturés et éventuellement d'autres coagents de réticulation, et avec addition d'agents générateurs de radicaux, selon le procédé d'une polymérisation en solution ou en suspension, pour former un hydrogel, et est fragmenté, séchée, broyée et tamisée.

**25.** Procédé suivant la revendication 24, caractérisé en ce qu'on traite les polymères avec au moins un agent de réticulation de surface et en ce qu'on effectue une réticulation de surface à une température élevée.

**26.** Procédé suivant la revendication 25, caractérisé en ce que le traitement de surface et la réticulation sont effectués à plusieurs reprises.

**27.** Utilisation des polymères suivant l'une des revendications 1 à 23, comme produits d'absorption d'eau et de liquides aqueux.

**28.** Utilisation de polymères suivant l'une des revendications 1 à 23, dans des constructions pour l'absorption de liquides corporels.

**29.** Utilisation de polymères suivant l'une des revendications 1 à 23, comme composants absorbant de l'eau ou des liquides aqueux dans des câbles conducteurs de courant ou de lumière, comme composants dans des matières d'emballage, comme produits d'amélioration de sol et dans la culture des plantes.

**30.** Utilisation de polymères suivant l'une des revendications 1 à 23, dans des articles plats, mousses ou non mousses, qui absorbent de l'eau ou des liquides aqueux.

**31.** Utilisation de polymères suivant l'une des revendications 1 à 23, comme substance de support d'engrais ou d'autres substances actives qui sont recédées à l'environnement d'une manière retardée pendant une période de temps prolongée.

**32.** Agents de réticulation suivant la formule générale I :

$$H_oR_3(NR_1R_2)_p$$

$R_1$, $R_2$ :     $CHR=CH-CH_2$,        H pour $o = 0$, $p = 2$

R :    H, $CH_3$        pour $o = 1$, $p = 1$

$R_3$ :   $R_a-C(H)_c(R_6)-CH_2$

$R_a$ :    monovalent pour $c = 0$ : H
divalent pour $c = 1$ : $CH_2$
$OCH_2$
$C_{1-6}-O-(CHR-CHR-O)_d$        $d = 1-45$
$C_{1-6}-CO-O(CHR-CHR-O)_d$

$R^6$ :    $R_7H$

$R_7$ :    O, $O(CHR-CHR-O)_n$        n : 1 à 45

à la condition qu'au moins deux radicaux $R_1$, $R_2$ représentent $CHR=CH-CH_2$.

**33.** Agents de réticulation de la formule générale II :

$$R_8(NR_1R_2)_2$$

$R_1$, $R_2$ :     $CHR=CH-CH_2$, H

R:    H, $CH_3$

$R_8$ :  $CH_2-CH(R_6)-CH_2-O-R_4-CH_2-CH(R_6)-CH_2$

$R_4$ :    $(CHR-CHR-O)_m$        m = 1 à 45
$C_1-C_6-O$
$R_5(O-R_3-NR_1R_2)_r-O$

r = 0 : lors de l'utilisation de composés diglycidiques
r = 1 : lors de l'utilisation de composés triglycidiques
r = 2 : lors de l'utilisation de composés tétraglycidiques,

$R_5$:    radical alkylène à base d'un polyol, éventuellement comportant une ou deux fonctions OH, qui peuvent éventuellement être mises à réagir avec jusqu'à 45 moles d'oxyde d'alkylène,
$R_3$ :    $CH_2-CH(R_6)-CH_2$

$R_6$ :    $R_7H$

$R_7$ :    O, $O(CHR-CHR-O)_n$        n = 1 à 45

à la condition qu'au moins deux radicaux $R_1$, $R_2$ représentent $CHR=CH-CH_2$.

**34.** Agents de réticulation de la formule générale III :

$$(R_9\text{-}R_3)_a N(B)_c (R_1)_j (R_2)_k$$

B : $C_1$-$C_6$-alkyle pour c = 0, on a :

$R_9$ : CHR=CH-CH$_2$-O pour a = 1, j = 1 et k = 1

CH$_2$=CR-CO-O pour a = 2, j = 1 et k = 0

$R_1$, $R_2$ : CHR=CH-CH$_2$, H pour a = 3, j et k = 0

R: H, CH$_3$

$R_3$ : CH$_2$-CH($R_6$)-CH$_2$ pour c = 1, on a :

$R_6$: $R_7$H pour a=1, j=1 et k=0

$R_7$ : O, O(CHR-CHR-O)$_n$ pour a = 2, j et k = 0

n : 1 à 45.

**35.** Agents de réticulation de la formule générale IV :

$$R_{11}[N(A)_b\text{-}(R_3\text{-}R_9)_p]_z$$

pour b = 1, p = 1

pour b = 0, p = 2

z=2,3,4

A : H, $C_1$-$C_6$-alkyle

$R_3$ : CH$_2$-CH($R_6$)-CH$_2$

$R_6$ : $R_7$H

$R_7$: O, O(CHR-CHR-O)$_n$ n : 1 à 45

R: H, CH$_3$

$R_9$ : CHR=CH-CH$_2$-O

CH$_2$=CR-CO-O

$R_{11}$ : radical alkylène di-, tri- ou tétravalent du composant aminé (en mélange ou isolément aliphatique, cycloaliphatique, aromatique, hétérocyclique).

**36.** Agents de réticulation suivant l'une des revendications 1 à 6, 10, 11 et 32 à 35, caractérisés en ce que les fonctions amine sont quaternisées.

**37.** Utilisation des composés suivant l'une des revendications 32 à 35 pour la réticulation et/ou la ramification de polymères en solution ou en masse.